# EUROPEAN PATENT SPECIFICATION

(11) **EP 1 993 483 B1**
(45) Date of publication and mention of the grant of the patent: **19.06.2013**
(21) Application number: 07752452.8
(22) Date of filing: 09.03.2007
(51) Int. Cl.: A61F 2/38

(54) **PROSTHETIC DEVICE AND METHOD FOR PLANNING THE IMPLANTATION**
PROTHESE SOWIE VERFAHREN ZUM PLANEN DER IMPLANTATION
DISPOSITIF PROTHETIQUE ET PROCEDE POUR PLANIFIER L'IMPLANTATION

(30) Priority: 13.03.2006 US 781867 P; 13.03.2006 US 781909 P; 13.03.2006 US 781910 P
(43) Date of publication of application: 26.11.2008
(73) Proprietor: MAKO SURGICAL CORP., Fort Lauderdale, FL 33317 (US)
(72) Inventor: HAJAJ, Binyamin, Plantation, FL 33322 (US); OTTO, Jason, K., Plantation, FL 33324 (US); ABOVITZ, Rony, Hollywood, FL 33021 (US); BROWN, Steven, B., Parkland, FL 33076 (US); BANKS, Scott, Gainesville, FL 32607 (US); FREGLY, Benjamin, J., Gainesville, FL 32608 (US); MEARS, Dana, C., Pittsburgh, PA 15215 (US)
(74) Representative: Lawrence, John
(86) International application number: PCT/US2007/005755
(87) International publication number: WO 2007/108933

(56) References cited:
- US-A1- 2004 236 424
- US-A1- 2005 154 471

## Description

### BACKGROUND OF THE INVENTION

### Field of the Invention

The invention relates to orthopedic joint replacement and, more particularly, to a prosthetic device for use in orthopedic joint replacement for resurfacing an articular surface of a bone.

### Description of Related Art

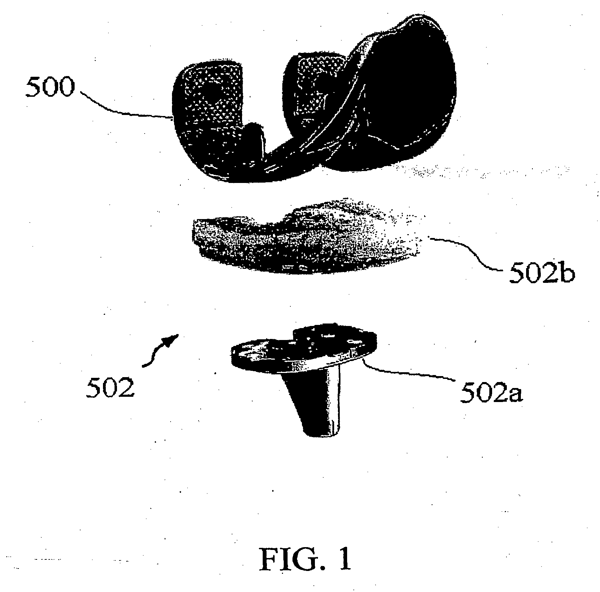

As shown in FIG. 1, conventional total knee arthroplasty (TKA) systems typically include a femoral component 500 that is implanted on the distal end of the femur and replaces the bearing surfaces of the femur, a tibial component 502 that is implanted on the proximal end of the tibia and replaces the bearing surfaces of the tibia and meniscus, and a patellar component (not shown) that replaces the articular surface of the patella. The femoral component 500 is typically a single solid component. The tibial component 502 may include a tibial baseplate (or tray) 502a that is affixed to the bone and a tibial insert 502b that is disposed on the tibial baseplate 502a and forms the bearing surfaces of the tibial component 502. Alternatively, the tibial bearing surface may be cemented directly to the bone. In operation, the bearing surfaces of the femoral component 500 articulate against the bearing surfaces of the tibial component 502 as the knee joint moves through a range of motion.

One disadvantage of conventional TKA systems is that the incision must be large enough to accept implantation of the femoral component 500 and the tibial component 502. Another disadvantage is that the femoral component 500 and the tibial component 502 have standard, fixed geometries and are available in a limited range of sizes. As a result, the surgeon may be unable to achieve a fit that addresses each patient's unique anatomy, ligament stability, and kinematics. Additionally, because conventional implant geometry is fixed, the surgeon may be forced to remove healthy as well as diseased bone to accommodate the implant. Thus, conventional TKA systems lack the flexibility to enable the surgeon to select implant components that are customized to accommodate a patient's unique anatomy and/or disease state.

In an effort to overcome disadvantages of conventional TKA systems, modular TKA knee prostheses comprising multiple components that are inserted separately and assembled within the surgical site have been developed. An example of a modular system is described in U.S. Patent Application Serial No. 11/312,741, filed December 30, 2005, published as Pub. No. US 2006/0190086. One disadvantage of such systems is that the modular components, although inserted separately, are connected together inside the patient's body. Thus, the modular components mimic a conventional TKA system, and, as a result, have limitations similar to those of a conventional TKA system. Additionally, because the modular components are fixed together, the components are dependent upon one another in that the selection and placement of one modular component is determined (or constrained by) the selection and placement of another modular component. For example, each modular component must include a connection mechanism (e.g., pins, screws, etc.) designed to mate with a corresponding connection mechanism on another modular component. Because the two components must mate together, the selection and placement of a component is determined and constrained by the selection and placement of the mating component. As a result, the degrees of freedom, interchangeability, and design variability of each modular component are restricted and the final geometry of the assembled component is fixed. Thus, conventional modular implants do not enable the surgeon to vary the placement or geometry of each modular component to best suit each patient's unique anatomy, ligament stability, kinematics, and disease state.

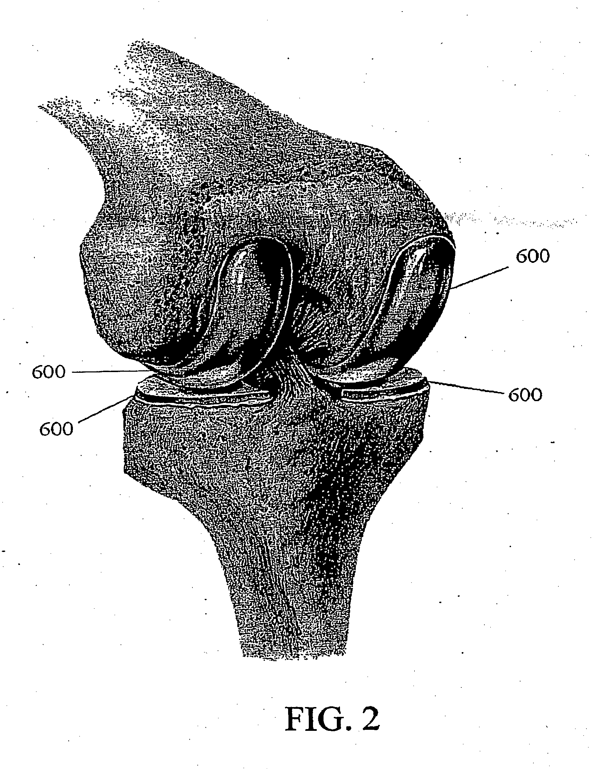

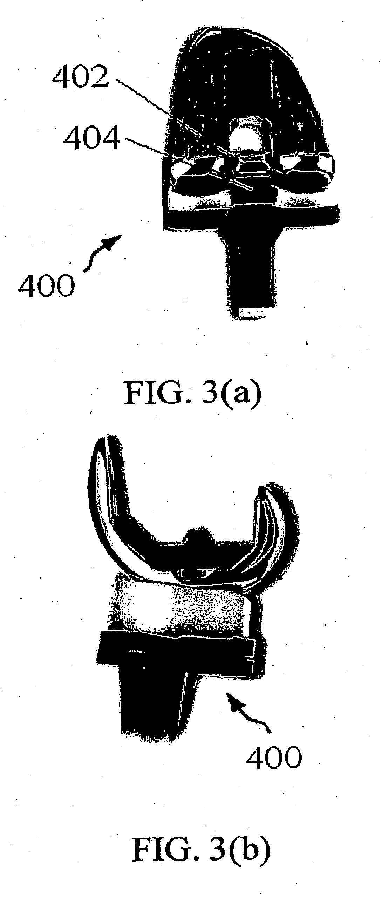

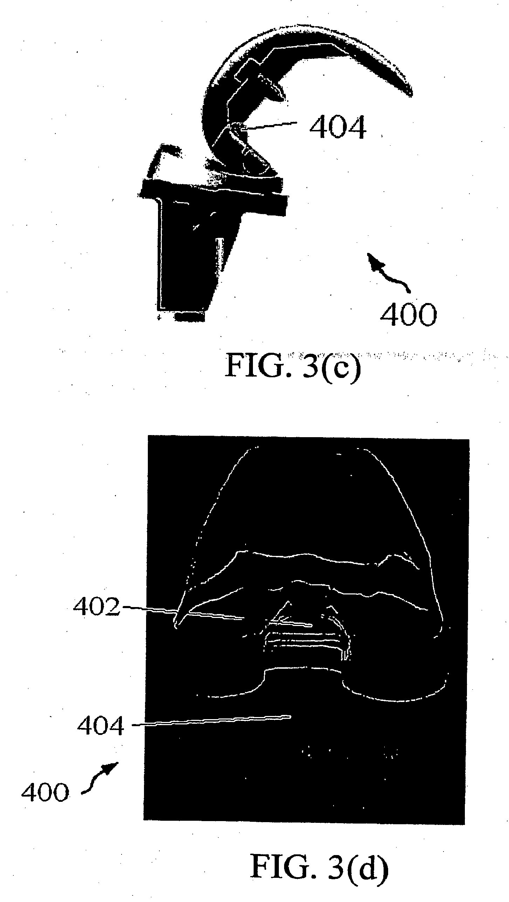

Conventional knee arthroplasty systems exist that include multiple unconnected components 600 (e.g., a bicondylar knee arthroplasty system as shown in FIG. 2), but such systems may only be able to address disease in two compartments of the knee - the medial compartment and the lateral compartment. Additionally, these systems are designed as non-constraining implants and thus are limited for use in patients with intact ligaments. As a result, such systems are unable to accommodate patients with disease that has progressed to the central (e.g., anterior) compartment of the femur or who have deficient ligaments. For example, when a patient has a deficient posterior cruciate ligament (PCL), the PCL may not be able to provide the necessary constraint to the joint. Thus, the PCL may need to be excised. In such situations, the functionality of the PCL (e.g., limiting translation of the femur on the surface of the tibia) may be provided by the introduction of a mechanical constraint via the implant. In conventional knee systems, this functionality is provided by a posterior stabilized (PS) implant, which is a TKA system that includes constraining elements in the central portion of the implant. For example, as shown in FIGS. 3(a)-3(c), a conventional PS implant 400 includes an aperture 402 in the central portion of the femoral component and a post 404 in the central portion of the tibial component. In operation, the aperture 402 receives the post 404 and restricts movement of the post 404 so that translation of the femur across the surface of the tibia is limited. Because conventional unconnected UKA systems only include components for the medial and lateral compartments of the knee, such implants are not suitable for requiring posterior stabilization or resurfacing of the central compartment of the knee.

Another disadvantage of such conventional systems is that the unconnected components 600 require accurate alignment relative to one another. A unicondylar implant (i.e., encompassing only a medial or a lateral compartment of the joint) may perform well because the biomechanics of the joint are not governed soley by the implant but also by the intact articular surfaces of the healthy condyle and by the intact ligaments. For a bicondylar implant (shown in FIG. 2), however, the implant encompasses both the medial and lateral compartments of the joint. As a result, the femorotibial joint is completely replaced. In order to maintain the natural kinematics of the joint and to work in conjunction with the intact ligaments, the components 600 must be aligned relative to one another and with the ligaments with a high degree of accuracy. Conventional freehand sculpting techniques, however, require a high degree of surgical skill and training and may not enable sufficient accuracy in a repeatable, predictable manner.

In view of the foregoing, a need exists for techniques and implants that enable individual components of a prosthetic device to be selected and implanted in one, two, or three compartments of a joint with a high degree of accuracy and in any combination that enables the surgeon to vary the geometry and configuration of the implant to create a customized prosthetic device tailored to the patient's unique anatomy, ligament stability, kinematics, and disease state.

U.S. Pub. No. 2005/0154471, which was published on July 14, 2005. describes a knee replacement system that includes a fermoral replacement, a tibial replacement, and a patellar replacement. The replacements can be segmented into components so that they can be used independently and/or jointly, and need not align with one another in a particular orientation. The cross-sections of the components can be formed in a variety of shapes, including the condyle areas of components.

U.S. Pub. No. 2004/0236424, which was published on November 25, 2004, describes methods, compositions, tools, and materials for repairing articulating slurfaces of a joint by replacing a portion of a joint with an implant material so that the joint achieves a near anatomic fit with surrounding structures and tissues.

### SUMMARY OF THE INVENTION

An aspect of the present invention relates to a method of planning implant placement of a prosthetic device configured to form at least a portion of a joint, as defined by appended claim 1. The method includes selecting a first component of the prosthetic device configured to be implanted in a body, determining a placement at which the first component will be fixed relative to a bone of the body, selecting a second component of the prosthetic device configured to be implanted in the body, and determining a placement at which the second component will be fixed relative to the bone. The determination of the placement of the second component is not constrained by a connection to the first component.

Another aspect of the present invention relates to a prosthetic device configured to form at least a portion of a joint, as defined by appended claim 3. The prosthetic device includes a plurality of components configured to be implanted in a body. Each of the plurality of components is configured to be fixed relative to a bone of the body. Each of the plurality of components is also configured such that a placement at which the component will be fixed relative to the bone is not constrained by a connection to another of the components

The prosthetic device may include a plurality of segmented components configured to form at least a portion of a joint, in whicheach of the plurality of segmented components is configured such that a placement of one of the segmented components in the joint is not constrained by a connection to another of the segmented components.

### BRIEF DESCRIPTION OF THE DRAWINGS

The accompanying drawings, which are incorporated in and constitute a part of this specification, illustrate embodiments of the invention and together with the description serve to explain principles of the invention.

FIG. 1 is a perspective view of a conventional total knee arthroplasty system.

FIG. 2 is a perspective view of a conventional bicondylar knee arthroplasty system.

FIGS. 3(a)-3(d) are perspective views of a conventional stabilized total knee arthroplasty system.

FIG. 4 is a coronal view of a knee joint.

FIG. 5(a) is a perspective view of an embodiment of a prosthetic device according to the present invention implanted in a knee joint.

FIG. 5(b) is a perspective view of an underside of the femoral components of the prosthetic device of FIG. 5(a).

FIG. 5(c) is a perspective view of the femoral components of the prosthetic device of FIG. 5(a) in a bicompartmental (medial and patellofemoral) configuration.

FIG. 5(d) is a perspective view of an embodiment of a prosthetic device according to the present invention.

FIG. 6 is a perspective view of an embodiment of a prosthetic device according to the present invention implanted in a knee joint.

FIG. 7(a) is a front perspective view of the prosthetic device of FIG. 6 with a knee joint in extension.

FIG. 7(b) is a side perspective view of the prosthetic device of FIG. 6 with the knee joint in extension.

FIG. 7(c) is a top perspective view of the prosthetic device of FIG. 6 with the knee joint in flexion.

FIG. 7(d) is a side perspective view of the prosthetic device of FIG. 6 with the knee joint in flexion.

FIG. 8 is a perspective view of a femoral component of an embodiment of a posterior stabilized prosthetic device according to the present invention.

FIGS. 9(a)-9(c) are perspective views of the component of FIG. 8a showing various fixation devices.

FIG. 10 is an illustration of a tibial component of an embodiment of a posterior stabilized prosthetic device according to the present invention.

FIG. 11 is an illustration of a femoral component of an embodiment of a prosthetic device according to the present invention.

FIG. 12 is an illustration of the sagittal, transverse, and coronal anatomical planes.

FIG. 13 is a cross-sectional sagittal view of a femur and a tibia of a knee joint.

FIG. 14 is a cross-sectional sagittal view of a conventional total knee arthroplasty system.

FIG. 15(a) is a cross-sectional sagittal view of a medial tibial component of an embodiment of a prosthetic device according to the present invention.

FIG. 15(b) is a cross-sectional sagittal view of a lateral tibial component of an embodiment of a prosthetic device according to the present invention.

FIG. 16 is a cross-sectional coronal view of a femoral component and a tibial component of an embodiment of a prosthetic device according to the present invention.

FIG. 17 is a cross-sectional sagittal view of a tibial component of an embodiment of a prosthetic device according to the present invention.

FIG. 18 is a cross-sectional coronal view of a tibial component of an embodiment of a prosthetic device according to the present invention.

FIG. 19 is a cross-sectional sagittal view of a tibial component of an embodiment of a prosthetic device according to the present invention llustrating a lowpoint located at an anterior-posterior midplane.

FIG. 20 is a cross-sectional sagittal view illustrating how lowpoints change as a slope of a medial tibial component and a lateral tibial component change according to an embodiment of the present invention.

FIGS. 21(a)-21(c) are cross-sectional sagittal views illustrating lowpoints of a medial tibial component and a lateral tibial component of an embodiment of a prosthetic device according to the present invention.

FIG. 22 is a cross-sectional coronal view of a medial tibial component and a lateral tibial component implanted on a proximal end of a tibia according to an embodiment of the present invention.

FIG. 23(a) is a cross-sectional sagittal view of medial and lateral tibial components of an embodiment of a prosthetic device according to the present invention illustrating degrees of freedom.

FIG. 23(b) is a top view of the tibial components of FIG. 23(a).

FIG. 24 is a perspective view of a haptic guidance system:

FIG. 25 is a view of a surgical navigation screen according to the present invention.

### DETAILED DESCRIPTION OF PREFERRED EMBODIMENTS

Presently preferred embodiments of the invention are illustrated in the drawings. An effort has been made to use the same or like reference numbers throughout the drawings to refer to the same or like parts.

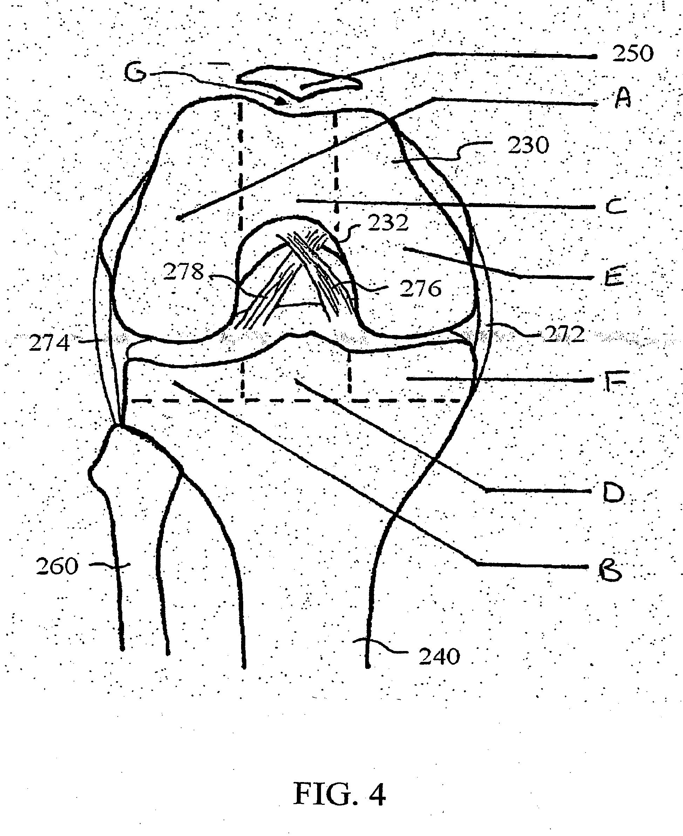

FIG. 4 is a diagram of a knee joint that includes a distal end of a femur 230, a proximal end of a tibia 240, a fibula 260, and a patella 250. The patella 250 moves relative to the femur 230 and the tibia 240 when the knee joint articulates. The femur 230 is joined to the tibia 240 by a medial collateral ligament (MCL) 272, a posterior cruciate ligament (PCL) 278, and an anterior cruciate ligament (ACL) 276. The femur 230 is joined to the fibula 260 by a lateral collateral ligament (LCL) 274.

The distal end of the femur 230 is conceptually divided into a lateral (i.e., outside) condyle region A, a central (or patellofemoral) region C (which contains a patellar groove 232 having an inverted U-shape), and a medial condyle (i.e., inside) region E. Similarly, the proximal end of the tibia 240 is conceptually divided into lateral B, central D, and medial F regions, which correspond, respectively, to the lateral A, central C, and medial E regions of the femur 230. Finally, the space between the patella 250 and the femur 230 or the tibia 240 (depending on the bending state of the leg) defines a patellar region G.

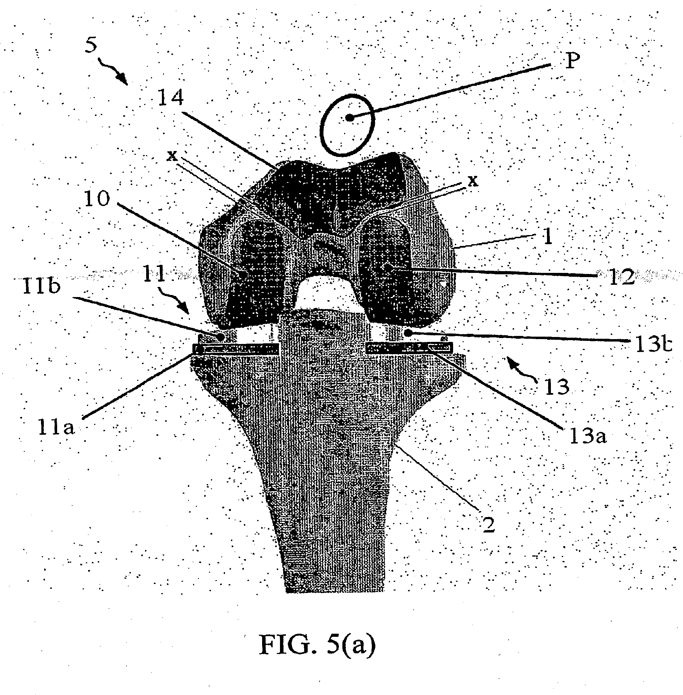

FIG. 5(a) shows an embodiment of a prosthetic device 5 according to the present invention. In this embodiment, the prosthetic device 5 is a knee implant. The present invention, however, is not limited to knee implants. The prosthetic device 5 may be any orthopedic joint implant, such as, for example, a total knee implant; a unicompartmental, bicompartmental, or tricompartmental knee implant; implants for other joints including hip, shoulder, elbow, wrist, ankle, and spine; and/or any other orthopedic and/or musculoskeletal implant, including implants of conventional materials and more exotic implants, such as orthobiologics, drug delivery implants, and cell delivery implants. In the alternative the prosthetic device may be a trial of an implant.

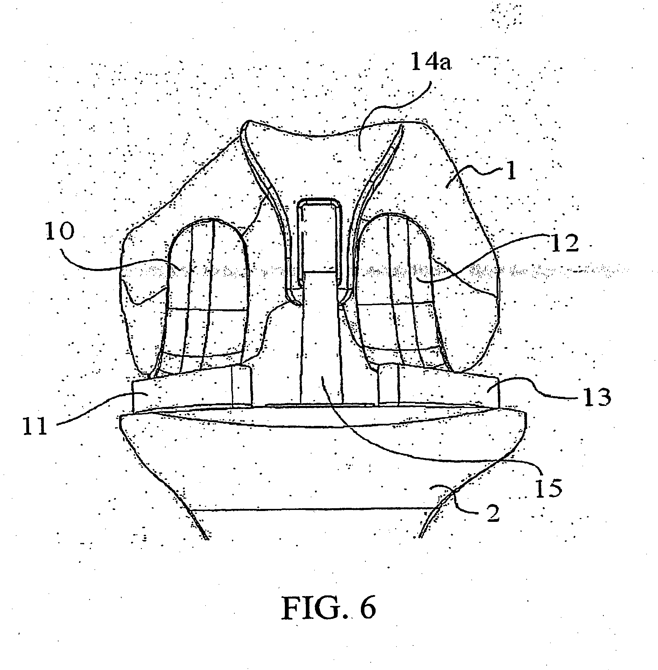

The prosthetic device 5 includes a plurality of components configured to be implanted in a body of a patient to form at least a portion of a joint, such as a knee joint as shown in FIG. 5(a). In this embodiment, the prosthetic device 5 includes a first component 10, a second component 12, and a third component 14 each configured to be fixed relative to a first bone 1 of the body. The prosthetic device 5 also includes a fourth component 11 and a fifth component 13 each configured to be fixed relative to a second bone 2 of the body. The prosthetic device 5 may also include additional components, such as a sixth component 15 configured to be fixed relative to the second bone 2, as shown in FIG. 6. In this embodiment, the components 10, 12, and 14 comprise femoral components, and the first bone 1 is a femur. The components 11 and 13 comprise tibial components, and the second bone 2 is a tibia.

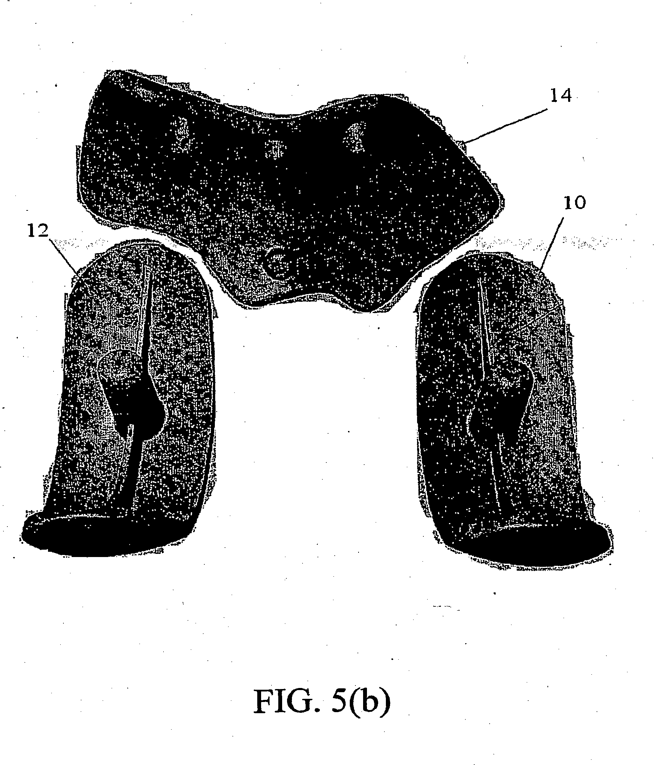

The components of the prosthetic device 5 are preferably segmented components. As shown in FIGS. 5(a) and 5(b), a segmented component is an individual component implanted in the joint as an independent, self-contained, stand-alone component that is not physically constrained by any other segmented component (as used herein, the term physically constrained means that the components are linked through a physical connection and/or physical contact in such a manner that the link between the components imposes limitations on the positioning or placement of either of the components). Thus, the components 10, 11, 12, 13, and 14 are all segmented components. Although a segmented component is an independent, stand-alone component, a segmented component itself may be formed by joining multiple components together (e.g., via mechanical joint, bonding, molding, etc.). For example, the segmented component 11 may be a medial tibial component formed by connecting a modular tibial baseplate 11a and a modular tibial insert 11b to form the independent, stand-alone medial tibial component 11. Although formed from multiple pieces, the tibial component 11 is a segmented component according to the present invention because, when implanted in the joint, it is not physically constrained by any other segmented component of the prosthetic device 5, such as the component 13 (shown in FIG. 5(a)) or the component 15 (shown in FIG. 6). To ensure that a segmented component is not physically constrained by other components, the segmented component may be implanted in the joint so that the component is not connected to and/or in contact with any other segmented component. For example, in one embodiment, the components of the prosthetic device 5 are configured such that the components can be implanted to form the prosthetic device 5 without being connected, as shown in FIGS. 5(a) and 6. In this embodiment, the components 10, 12, and 14 are not interconnected when fixed relative to the first bone 1. Similarly, the components 11, 13, and 15 are not interconnected when fixed relative to the second bone 2. In another embodiment, the components of the prosthetic device 5 are configured such that the components can be implanted to form the prosthetic device 5 without being in contact, as shown in FIGS. 5(a) and 6. In this embodiment, the components 10, 12, and 14 are physically separated from one another when fixed relative to the first bone 1. Similarly, the components 11, 13, and 15 are physically separated from one another when fixed relative to the second bone 2.

One advantage of a prosthetic device having unconnected and/or physically separated components is that the surgeon does not have to consider whether a particular component is designed to mate with other components of the prosthetic device 5. Instead, the surgeon can select each component based on how that particular component will fit to the specific patient anatomy and the expected performance in the specific region of the joint in which it will be implanted. As a result, the surgeon can create a customized prosthetic device, for example, by selecting each component to have the performance characteristics (e.g., size, geometry, conformity, orientation, angle, etc.) best suited for the particular portion of the joint in which it will be installed. In contrast, with conventional modular implants, the surgeon must use modular components that have corresponding connection mechanisms. Thus, the surgeon may be limited to the implant manufacturer's predetermined component combinations and/or forced to select components having less desirable performance characteristics just to ensure that the components can be successfully mated together.

Another advantage of a prosthetic device having unconnected and/or physically separated components is that the position of each component on the bone is not constrained or hindered by the position of any other component on the bone. Thus, a pose (i.e., position and orientation) or placement at which each component is fixed relative to the bone is not constrained by a connection to or contact with another component. As a result, the degrees of freedom available when positioning a component are not limited or restricted by any other component. As a result, the surgeon has freedom to customize the placement (e.g., alignment, orientation, rotation, translation, etc.) of each component of the prosthetic device 5 to meet the specific needs of the patient (e.g., based on unique anatomy, ligament stability, kinematics, and/or disease state). In contrast, conventional TKA implants include monolithic components having fixed geometry. Similarly, conventional modular implants include modular pieces that are fixed together after insertion into the body resulting in fixed geometry. Because the geometry is fixed, the surgeon does not have the freedom to independently position each modular piece.

Another advantage of a prosthetic device having unconnected and/or physically separated components is that the configuration of the prosthetic device 5 is variable. For example, because the components do not constrain one another, the combinations of components forming the prosthetic device 5 can be varied (e.g., mixed and matched) to include any number, type, and/or combination of components appropriate for a particular patient. The appropriate number, type, and/or combination of components may be determined based on patient specific factors such as, for example, the patient's unique anatomy, ligament stability, kinematics, and/or disease state. Thus, by varying the number, type, and/or combination of components, the surgeon can customize the prosthetic device 5 to target osteoarthritic disease by joint compartment. In contrast, with conventional TKA systems, there are typically up to eight different implant sizes offered for each component, and the average size increment is between 3-5 mm. These implants may have a fixed ratio between the anterior-posterior and the medial-lateral dimensions with other implant geometry being accordingly constrained. Because each patient's bone generally does not perfectly match the TKA implant size offering, the surgeon must compromise by downsizing or upsizing the component. Additionally, conventional TKA designs require the removal of a significant amount of bone to eliminate variations in the patient's joint geometry and ensure that one of the available implants will fit. As a result, ligament balance could be slightly looser or tighter than desired, or certain compartments could be overstuffed (i.e., more metal or plastic added than bone removed). In addition, the generally symmetric condyles of the femoral TKA component and the generally symmetric condyles of the tibial TKA component may not perfectly fit the patient's natural asymmetric anatomy. Another problem is that the kinematics of the joint following a TKA procedure are typically different from the natural kinematics. Thus, although the patient experiences significant improvement (e.g., reduced pain, increased range of motion, etc.), full function of the joint is not restored. In contrast, the present invention advantageously provides a segmented implant system with components having multiple sizes, shapes, geometries, and conformities to enable construction of a prosthetic device 5 customized to a particular patient's unique anatomy, ligament stability, kinematics, and/or disease state.

In operation, to target a patient's unique disease state, the surgeon can configure the prosthetic device 5 to address disease in any compartment of the joint. Specifically, the surgeon can mix and match the components of the prosthetic device 5 to provide the desired coverage. For example, the prosthetic device 5 may include components configured for implantation on a first compartment of a knee joint (e.g., a medial compartment), components configured for implantation on a second compartment of the knee joint (e.g., a lateral compartment), and/or components configured for implantation on a third compartment of the knee joint (e.g., a central compartment). As a result, the prosthetic device 5 can be configured as a unicompartmental, bicompartmental, or tricompartmental implant. Thus, the surgeon can vary an arrangement of the components to form a prosthetic device customized to the patient's unique anatomy, disease state, ligament stability, and kinematics.

In one embodiment, the components of the prosthetic device 5 are configured to form a tricompartmental implant. In this embodiment, the prosthetic device 5 includes at least three segmented components each configured to be fixed relative to a corresponding bone of the joint. The tricompartmental implant may be cruciate retaining (shown in FIG. 5(a)) for patients whose posterior cruciate ligament (PCL) and anterior cruciate ligament (ACL) are healthy and intact or posterior stabilized (shown in FIG. 6) for patients whose PCL is damaged and/or must be excised. In the cruciate retaining embodiment of FIG. 5(a), the components 10, 12, and 14 form a femoral portion of the tricompartmental implant, and the components 11 and 13 form a tibial portion of the tricompartmental implant. For the femoral portion, the component 10 may be a medial femoral component configured to be fixed relative to the medial femoral region E of the first bone 1, the component 12 may be a lateral femoral component configured to be fixed relative to the lateral femoral region A of the first bone 1, and the component 14 may be a patellofemoral component configured to be fixed relative to the central femoral region C of the first bone 1. For the tibial portion, the component 11 may be a medial tibial component (e.g., including a baseplate 11a and an insert 11b) configured to be fixed relative to the medial tibial region F of the second bone 2, and the and the component 13 may be a lateral tibial component (e.g., including a baseplate 13a and an insert 13b) configured to be fixed relative to the lateral tibial region B of the second bone 2. The prosthetic device 5 may also include a patella component P.

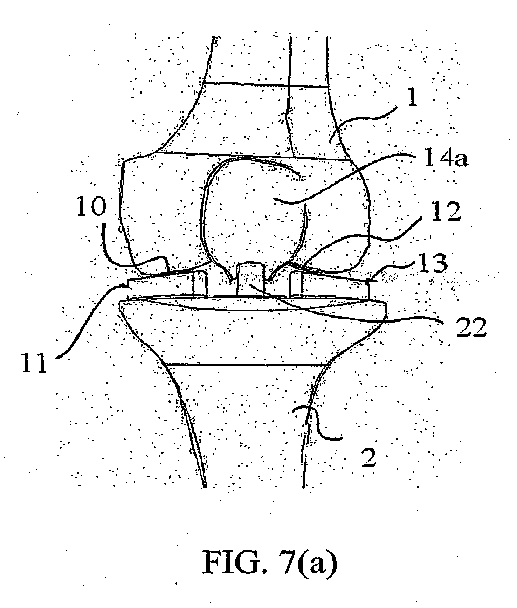

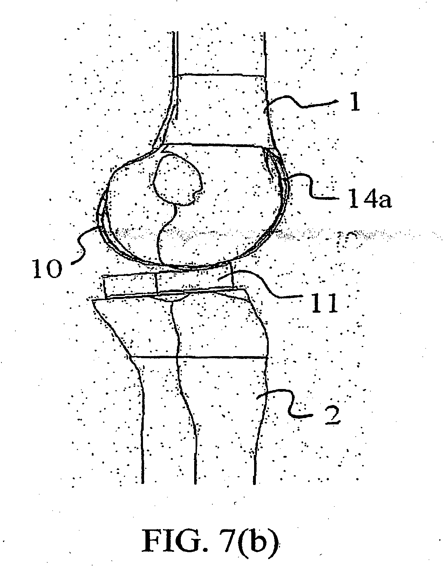

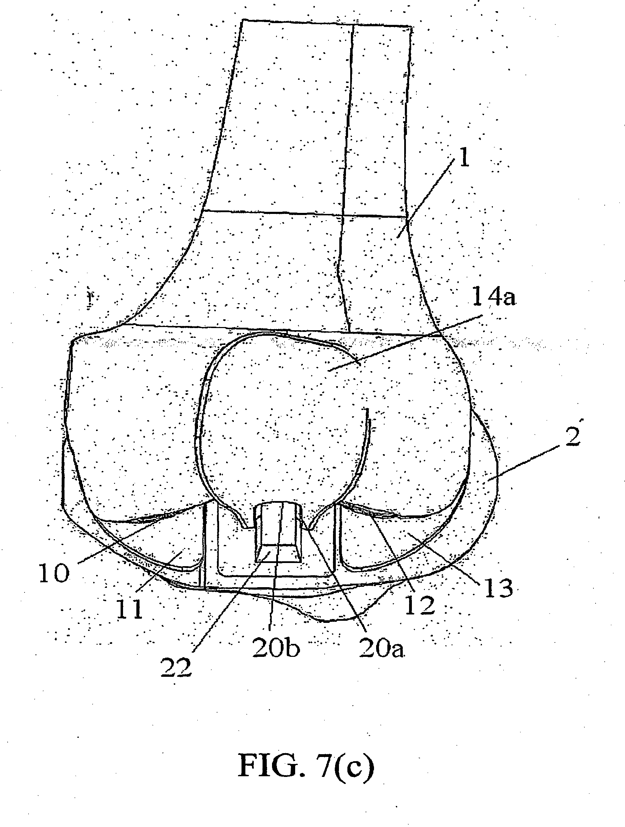

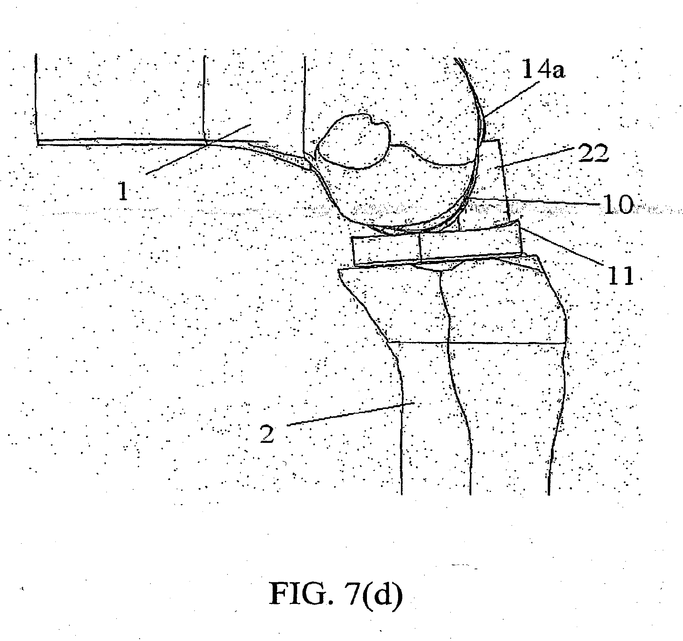

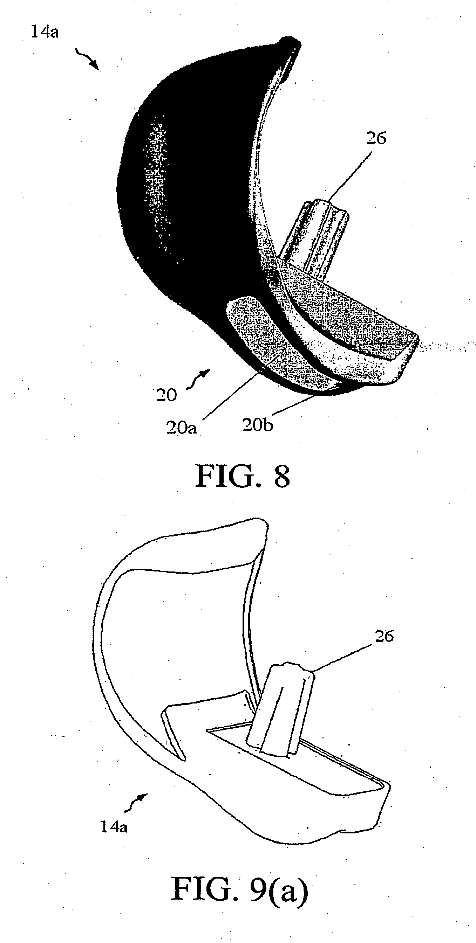

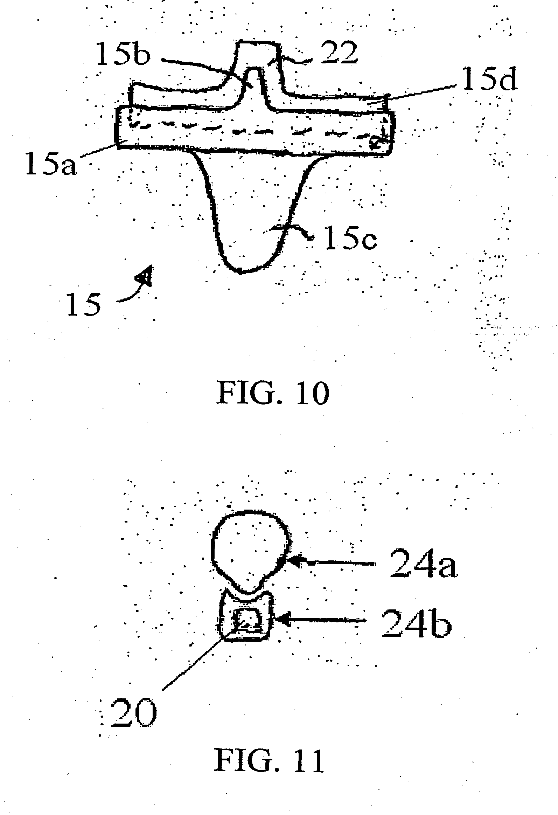

The tricompartmental cruciate retaining embodiment of FIG. 5(a) may be easily converted to a tricompartmental posterior stabilized embodiment by adding the component 15 and replacing the component 14 with the component 14a as shown in FIGS. 6 and 7(a) to 7(d). The component 14a is a patellofemoral component configured to be fixed relative to the central femoral region C of the first bone 1, and the component 15 is a central tibial component configured to be fixed relative to the central tibial region D of the second bone 2. In operation, the components 14a and 15, interact to replace the functionality of the excised PCL by imparting constraining forces that are absent in the natural joint due to deficient ligaments. Thus, the components 14a and 15 comprise a constraint mechanism. The constraint mechanism may be any suitable constraint mechanism, such as any constraint mechanism used in a conventional PS implant. In one embodiment, the component 14a includes a feature 20 (shown in FIG. 8) for constraining a portion of the tibial component 15, and the component 15 includes a corresponding feature 22 (shown in FIG. 10) that engages the feature 20. In a preferred embodiment, the feature 20 comprises a recess 20a and a stop member 20b. The stop member 20b may be, for example, a cam comprised of one or more internal surfaces of the recess 20a and functioning as a rigid restraint. For example, the stop member 20b may include an anterior, posterior, medial, and/or lateral surface of the recess 20a. The feature 22 includes a projection (e.g., a post or spine) as shown in FIG. 10 that is received in the recess 20a of the component 14a as shown in FIGS. 6, 7(a), and 7(c). In one embodiment, a clearance between a surface of the recess 20a and a surface of the feature 22 is between about 0.5 mm to about 1.5 mm. In operation, as the knee joint moves through a range of motion and the components 14a and 15 articulate, the feature 22 (on the tibial component 15) moves in the recess 20a (of the femoral component 14a) and contacts and is restrained by the stop member 20b. For example, an anterior, posterior, medial, and/or lateral region of the feature 22 may contact and be restrained by one or more surfaces of the recess 20a. As a result, movement (e.g., anterior-posterior, medial-lateral) of the component 15 relative to at least a portion of the component 14a is constrained. In this manner, the features 20 and 22 interact to generate constraining forces in the joint that mimic the functionality of the excised PCL.

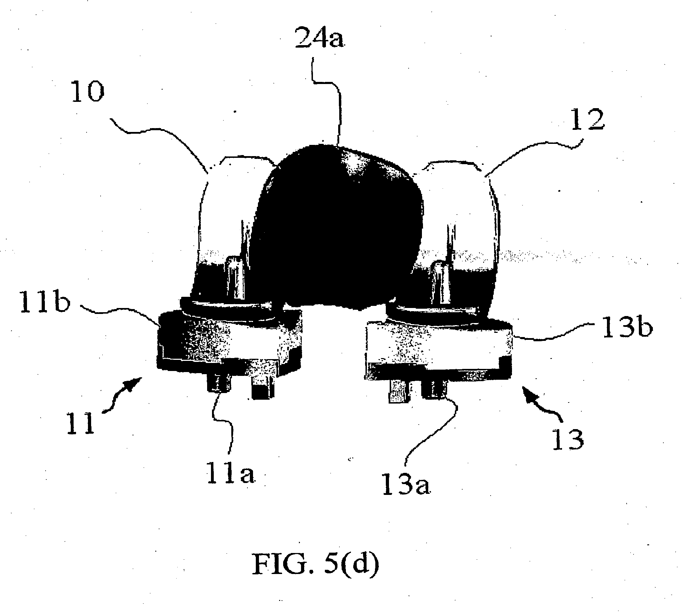

As with other components of the prosthetic device 5, the component 15 may be made of one or more pieces. In one embodiment, as shown in FIG. 10, the component 15 includes a tray 15a (having a post 15b and a stem 15c) and an insert 15d that may be affixed to the tray 15a in any known manner such as, for example, a snap fit or mechanical fastener. Similarly, the component 14a may include multiple pieces. For example, in one embodiment, the component 14a may include a first part 24a and a second part 24b as shown in FIG. 11. In this embodiment the first part 24a may be a patellofemoral component suitable for use in a cruciate retaining implant, such as the implant shown in FIG. 5(a). To create a cruciate retaining implant that addresses disease in the central compartment of the joint, the surgeon can implant only the first part 24a of the patellofemoral component in the central femoral region C of the first bone 1 as shown in FIG. 5(d). This cruciate retaining implant can easily be converted to a posterior stabilized implant by simply adding the second part 24b to the central femoral region C of the first bone 1 and the component 15 to the central tibial region D of the second bone 2. To provide posterior stabilization, the second part 24b of the patellofemoral component may include the feature 20 (shown in FIG. 8) that engages the feature 22 of the component 15 to generate constraint forces that mimic the functionality of the excised PCL.

Structurally, the parts 24a and 24b may be connected to form a single segmented component 14a. Alternatively, the parts 24a and 24b may be individual segmented components that are not connected to and/or not in contact with any other component of the prosthetic device 5 when implanted in the joint. For example, in one embodiment, the first and second parts 24a and 24b may be configured such that a placement at which one of the first and second parts 24a and 24b will be fixed relative to the central region C of the first bone 1 is not constrained by a connection to the other of the first and second parts 24a and 24b. In this embodiment, as shown in FIG. 11, the first and second parts 24a and 24b are not connected and do not include features for joining the first part 24a and the second part 24b.

One advantage of the posterior stabilized embodiment of the present invention is that the patellofemoral component (e.g., the component 14a, the first and second parts 24a and 24b) is a segmented component that is independent of the medial and lateral femoral components 10 and 12. Similarly, the central tibial component 15 is a segmented component that is independent of the medial and lateral tibial components 11 and 13. As a result, the posterior stabilized patellofemoral component and the central tibial component can be used alone to address disease in the central compartment of the joint or in combination with the medial and/or lateral components of the prosthetic device 5. Thus, the surgeon can vary the combination of components to form an implant customized to the patient's unique anatomy, disease state, ligament stability, and kinematics. In contrast, a conventional PS implant (shown in FIGS. 3(a)-3(d)) is available only as a TKA system with femoral and tibial components each having invariable fixed geometry and covering, respectively, an entire distal surface of the femur and an entire proximal surface of the tibia.

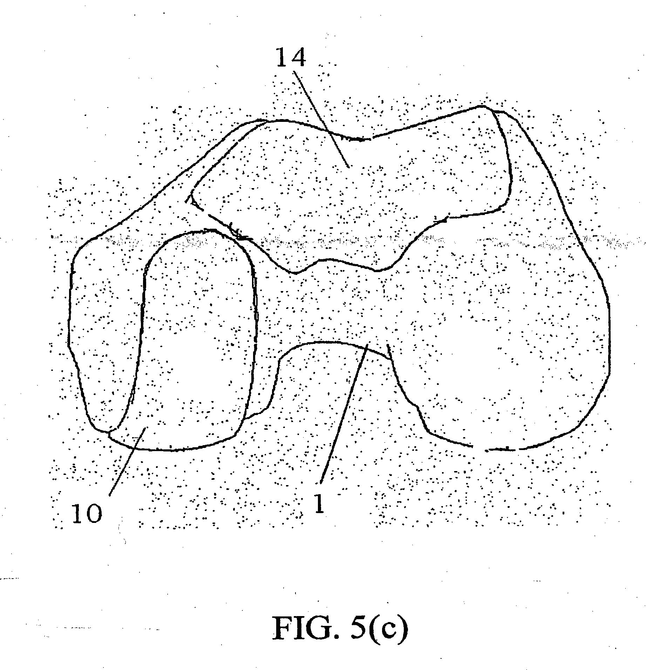

In another embodiment, the components of the prosthetic device 5 are configured to form a unicompartmental implant. For example, in reference to FIGS. 5(a) and 11, for a cruciate retaining embodiment, a unicompartmental implant may be formed by including only (a) the components 10 and 11 (medial compartment), (b) the components 12 and 13 (lateral compartment), (c) the component 14 (central compartment), or (d) the first part 24a (central compartment). Additionally, if the patella P has significant osteoarthritis, the surgeon may decide to resurface the patella P. In such cases, (c) and (d) may include a patellar component. Because the components are segmented, the unicompartmental embodiment can easily be converted into a bicompartmental or tricompartmental embodiment. For example, a bicompartmental implant may be formed by combining any two of (a), (b), and (c) or (d) above. For example, FIG. 5(c) illustrates a femoral portion of a bicompartmental implant that is a combination of (a) and (c). Similarly, a tricompartmental implant may be formed by combining three of (a), (b), and (c) or (d) above. For example, FIG. 5(a) illustrates a tricompartmental implant that is a combination of (a), (b), and (c).

Similarly, in reference to FIGS. 6 and 11, for a posterior stabilized embodiment, a unicompartmental implant can be formed by including only (e) the components 10 and 11 (medial compartment), (f) the components 12 and 13 (lateral compartment), (g) the components 14a and 15 (central compartment), or (h) the first part 24a, the second part 24b, and the component 15 (central compartment). Additionally, if the patella P has significant osteoarthritis, the surgeon may decide to resurface the patella P. In such cases, (g) and (h) may include a patellar component. Because the components are segmented, the unicompartmental embodiment can easily be converted into a bicompartinental or tricompartmental embodiment. For example, a bicompartmental implant may be formed by combining any two of (e), (f), and (g) or (h) above. Similarly, a tricompartmental implant may be formed by combining three of (e), (f), and (g) or (h) above. For example, FIG. 6 illustrates a tricompartmental implant that is a combination of (e), (f) and (g).

In one embodiment, the prosthetic device 5 is a bicompartmental implant that includes a first segmented component configured to be fixed relative to a central portion of a bone (e.g., a femur or a tibia) of the joint and a second segmented component configured to be fixed relative to at least one of a medial portion and a lateral portion of the bone. Thus, in this embodiment, the prosthetic device 5 encompasses the central compartments of the joint and either the medial or lateral compartment of the joint. For example, for a femoral portion of a cruciate retaining embodiment, the components 10 and 14 may be implanted on the first bone 1 (as shown in FIG. 5(c)), and the component 12 may be omitted. Similarly, for a tibial portion of a posterior stabilized embodiment; the components 11 and 15 may be implanted on the second bone 2 (as shown in FIG. 6), and the component 13 may be omitted.

The components of the prosthetic device 5 may be made of any material or combination of materials suitable for use in an orthopedic implant. Suitable materials include, for example, biocompatible metals (e.g., a cobalt-chromium alloy, a titanium alloy, or stainless steel); ceramics (e.g., an alumina or zirconia-based ceramic); high performance polymers (e.g., ultra-high molecular weight polyethylene); a low friction, low wear polymer/polymer composite; and/or a polymer composite as described in U.S. Patent Application No. 10/914,615, U.S. Patent Application No. 11/140,775, and/or International Application No. PCT/US2005/028234 (International Pub. No. WO 2006/020619).

The components of the prosthetic device 5 may be implanted in the joint in any known manner, for example, using an adhesive, a cement, an intramedullary rod, a press fit, a mechanical fastener, a projection (e.g., stem, post, spike), and the like. Fixation may also be accomplished via biological or bone in-growth. To promote biological in-growth, the components of the prosthetic device 5 may be coated with hydroxyapatite (HA), have a porous texture (e.g., beads, etc.), include one or more surfaces made from a porous metal (e.g., TRABECULAR METAL™ currently produced by Zimmer, Inc.), and/or include one or more surfaces having a cellular engineered structure (e.g., TRABECULITE™ currently produced by Tecomet). In one embodiment, each component of the prosthetic device 5 is implanted using the fixation device best suited for the compartment in which the component will be implanted. For example, the fixation device for a particular component may be selected based on bone quality at the specific site of implantation. For example, if the implantation site has a dense healthy bone, the surgeon may select an implant with a porous coating or porous metal to allow for bone in-growth fixation. The selection of one fixation device or method for one compartment of the joint does not determine the fixation device or method for another compartment. Thus, the components of the prosthetic device 5 may be implanted with similar or different fixation methods and devices.

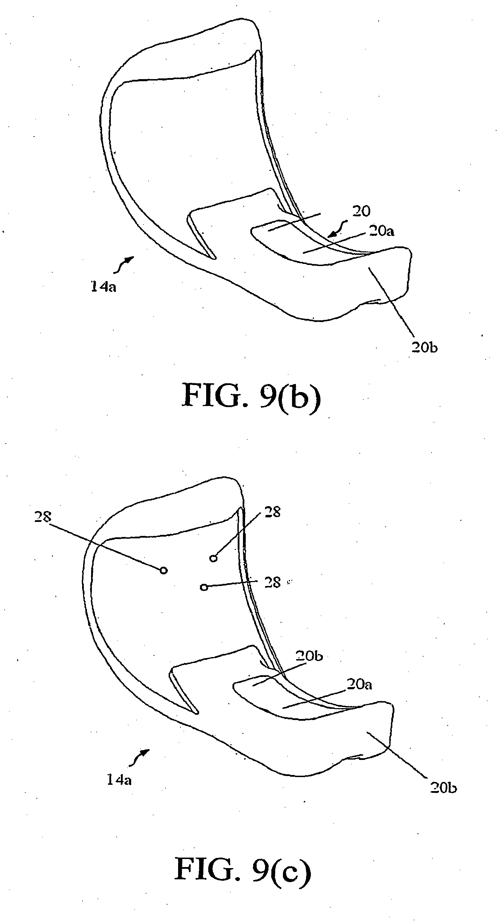

In one embodiment, the prosthetic device 5 includes a fixation device configured to be inserted into an intramedullary canal of a bone. For example, the component may include a projection or intramedullary canal fixation post 26 as shown in FIGS. 8 and 9(a) for the femur and a similar post on the corresponding tibial compoment. In another embodiment, as shown in FIG. 9(c), a fixation device includes surface features 28 (e.g., projections, posts, fasteners, spikes, biological in-growth sites, etc.) that promote fixation of the component to the bone. In another embodiment, the components of the prosthetic device 5 are configured to be affixed only to an anatomy of the patient (e.g., via press fit, mechanical fastener, adhesive, intramedullary rod, etc.) and not to other components of the prosthetic device 5. In this embodiment, each component lacks a feature (e.g., a pin, screw, mounting hole, dovetail joint, etc.) for joining the component to another component. As a result, the placement of a component is not is not constrained by a connection to another component. In yet another embodiment, the prosthetic device 5 includes a component configured to be press fit onto the bone. For example, the component may have a geometry (shown in FIG. 9(b)) that corresponds to a geometry of a corresponding surface on the bone. As a result, the component can be press fit to the bone. The corresponding surface on the bone may be, for example, a robotically prepared surface having tolerances engineered to permit the component to be press fit to the surface. The surface may be prepared, for example, as described in U.S. Patent Application Serial No. 11/357,197, filed February 21, 2006, published as Pub. No. US 2006/0142657.

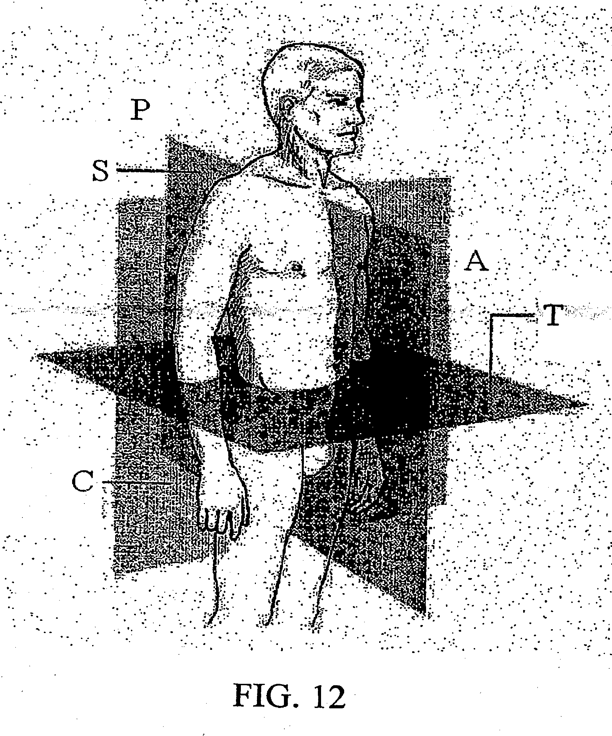

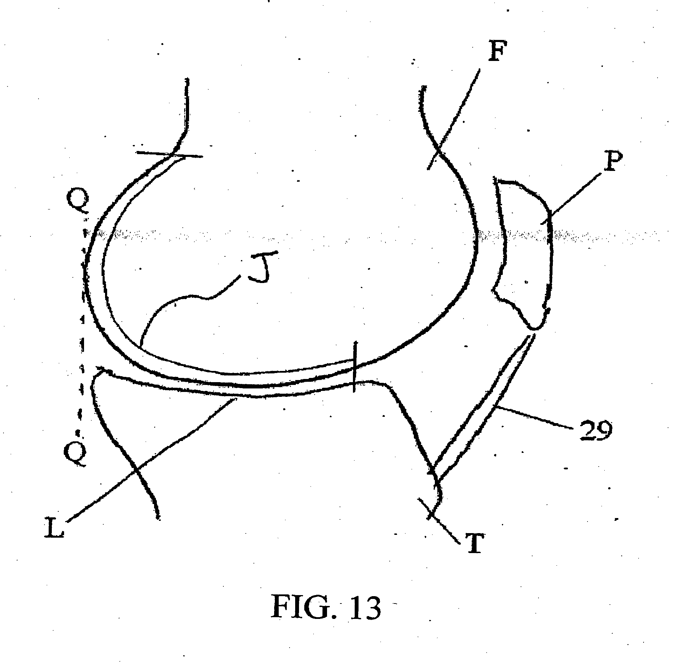

As shown in FIG. 12, anatomical planes of the body include a sagittal plane S, a transverse plane T, and a coronal plane C. The front of the body is known as anterior, and the back of the body is known as posterior. Thus, the sagittal plane S is an anterior-posterior (AP) plane. In a knee joint, the medial condyle of the femur F has a different sagittal geometry than the lateral condyle of the femur F. The sagittal shape of the femur F is commonly known as the j-curve because it is made of several arcs of varying radii, larger distally and smaller posteriorly, whose silhouette resembles the shape of a "J" as shown in FIG. 13. The radii of the medial and lateral arcs are different, and the angle at which the radii transition from one arc to the next also varies. Similarly, the sagittal cross-sectional shape of the medial tibial plateau is different from the sagittal cross-sectional shape of the lateral tibial plateau. The medial tibial side is generally described as more concave (or cup shaped or conforming). Conversely, the lateral tibial side is commonly described as convex (or flat or non-conforming). These shape differences between the medial and lateral sides of the femur F and the tibia T affect the net normal force of the contact region. For example, when contact vectors between the medial and lateral sides are not parallel, a moment develops between compartments, including an axial rotation moment that imparts axial rotation between the femur F and the tibia T. Additionally, these differences in shape of the articular surfaces of the tibia enable kinematics of the joint throughout the range of motion, including rotation, translation of the bones, and internal rotation that occurs during the gait cycle. In contrast, most conventional TKA systems have either symmetric or mirror-image sagittal shapes for the medial and lateral compartments of the femur F and the tibia T. Typically, in contrast to the natural geometry of the joint, the tibial shape of the implant is concave for both compartments. As a result, axial rotation between the femur F and the tibia T is restricted and abnormal knee kinematics may result.

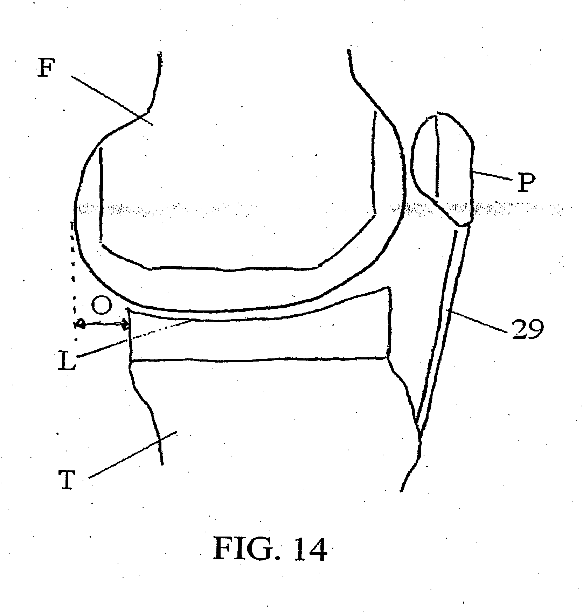

Additionally, in a natural joint, the sagittal shape of the medial tibial plateau has a lowpoint or sulcus L located at approximately a midpoint of the plateau in an anterior-posterior (front-back) direction. At full extension, the femur F rests in the sulcus L as shown in FIG. 13. When viewed in the sagittal plane S, the posterior femoral condyle is nearly flush with the posterior tibia T as indicated by a line Q-Q in FIG. 13. At this position, the anterior femur F is more anterior than the tibia T. As a result a patellar ligament 29 is directed anteriorly. During flexion, a force develops in the patellar ligament 29 due to quadriceps activation and causes the tibia T to translate anteriorly or the femur F to translate posteriorly. This is known as femoral rollback. In contrast to natural joint geometry, in a conventional TKA system, the tibial medial sagittal lowpoint L is located in the posterior one-third region of the tibial plateau. At full extension, the femur F rests in the sulcus L, which causes the posterior femoral condyle to overhang the tibia T posteriorly by an amount O as shown in FIG. 14. At this position, the anterior femur F is nearly flush with the anterior tibia T so the patellar ligament 29 is directed nearly vertically. During flexion, the patella P quickly translates posteriorly due to femoral shape. Accordingly, the patellar ligament 29 is directed posteriorly. The resulting force causes the tibia T to translate posteriorly or the femur F to translate anteriorly. This is known as paradoxical motion. Thus, the position of the tibial sagittal lowpoint L can affect knee motion or kinematics. Depending on ligament stability, the lowpoint L may need to be adjusted to provide appropriate knee kinematics for a particular patient.

Advantageously, the present invention can be adapted to address these problems. For example, the ability to select from a variety of segmented components, to mix and match the components, and to place the components as desired (i.e., without physical constraints imposed by other components), the surgeon can configure the prosthetic device 5 to correspond to the natural geometry of a healthy joint so that the resulting knee kinematics more closely mirror normal joint motion. Thus, rather than a limited number of components available in fixed configurations as with conventional TKA and connected modular implant systems, a variety of segmented components (e.g., of various sizes, geometries, conformities, etc.) can be designed and varied by the surgeon to create a prosthetic device having a precise fit for each patient.

For example, the components of the prosthetic device can be configured such that at least one of a geometry, a conformity, and a configuration of the prosthetic device 5 can be varied during implantation by varying at least one of a placement and a selection of one or more of the components. Because the components are unconnected and/or not in contact with one another, constraints on the surgeon's ability to select and place the components as desired are reduced. Thus, selection parameters (e.g., size, shape, geometry, conformity) and placement parameters (e.g., orientation, position, alignment) of one component are not determinative of the selection and/or placement parameters of another component during implantation (as used herein, the term determinative means that the selection or placement parameters of one component necessarily require particular selection or placement parameters of another component). As a result, the surgeon can alter the geometry, conformity, and/or configuration of the prosthetic device 5 to meet the customized needs of the patient by varying the components he selects and/or his placement of those components. As a result, the selection and placement of each component can be tailored to create a customized prosthetic device 5 that meets the patient's unique needs in each region of the joint.

With regard to placement, each component can be implanted in the joint with the orientation, position, and alignment best suited to the patient's unique anatomy, ligament stability, kinematics, and/or disease state. For example, in one embodiment, the components of the prosthetic device 5 may include a first component and a second component configured to be positioned relative to the bone such that an alignment of the first component is not determinative of an alignment of the second component during implantation. For example, during implant planning and placement, the component 10 (shown in FIGS. 5(a) and 5(b)) can be aligned based on the patient's needs in the medial compartment of the joint. Similarly, the components 12 and 14 can be aligned based on the patients needs in the lateral and central compartments, respectively. Because the components are segmented, each can be independently aligned. As a result, the alignment of one component does not depend on and is not constrained by the alignment of another component. Accordingly, during implant planning and placement, the surgeon has the freedom to vary the alignment and other placement parameters of each component to best suit the needs of the patient in the area of the joint where the component is being implanted. In this manner, the implanted components of the prosthetic device 5 enable optimal restoration of joint kinematics based on patient anatomy and previous joint function. Additionally, in situations where the patient has an existing deformity that requires surgical intervention and correction through implants, the ability to align components as desired enables optimal balancing of the joint after deformity correction.

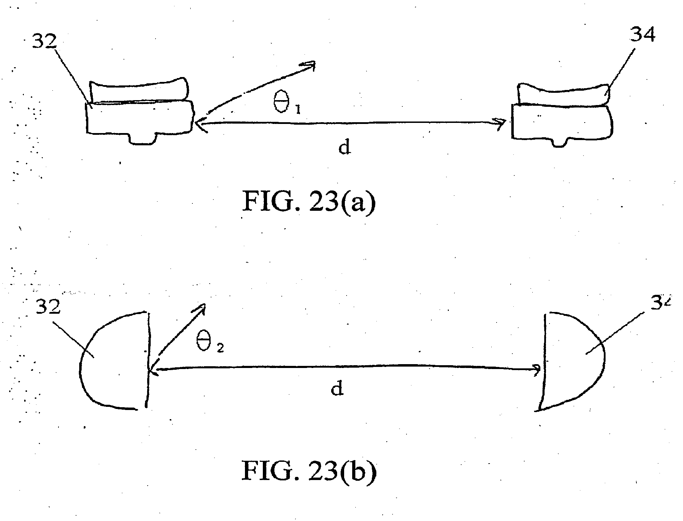

In one embodiment; the degrees of freedom of a first component of the prosthetic device are not determinative of the degrees of freedom of a second component of the prosthetic device. As a result, the surgeon has maximum flexibility when planning implant placement and when installing each component of the prosthetic device 5 in the joint. Because the components of the prosthetic device 5 are not connected to and/or in contact with other components of the prosthetic device 5 when implanted in the joint, each component can be independently positioned in one or more degrees of freedom. In a preferred embodiment, the components can be independently positioned in six degrees of freedom. For example, as shown in FIGS 23(a) and 23(b), the medial tibial component 32 can be oriented independently of the lateral tibial component 34 by an angle θ1 and an angle θ2. The distance d between the medial and lateral components 32 and 34 can also be adjusted. The medial and tibial components can be independently positioned with potentially different placements in the anterior-posterior, medial-lateral, and superior-inferior directions. Similarly, the components can be oriented with potentially different rotations in varus/valgus, internal/external, and flexion/extension (or posterior slope). The ability to vary the distance d between the components enables adjustment to unique patient geometry, or even to account for variations existing between male and female morphology, as well as between different populations (e.g., Asian, European, African, and others). The slope of the components defined by the angles θ1 and θ2 may be used by the surgeon to adjust the implant slope to an angle that he believes will result in better implant stability and or life depending on the existing precondition of ligaments.

Although FIGS. 23(a) and 23(b) illustrate tibial components, femoral components of the prosthetic device 5 can also be independently positioned in one or more (e.g., six) degrees of freedom. In one embodiment, a distance x (shown in FIG. 5(a)) between the patellofemoral component 14 and the medial component 10 or the lateral component 12 is less than or equal to about 5 mm. When the distance x (or gap) between the components is greater than 5 mm the patella may slip off of the component 14 into the gap and then pop onto the component 10 or 12 rather than smoothly transitioning from one component to another.

With regard to selection, each component can be selected to have the size, shape, geometry, and conformity best suited to the patient's unique anatomy, ligament stability, kinematics, and/or disease state and based on the surgical outcome desired by the surgeon for the patient. Conformity refers to the fit between components, such as the manner in which an articular surface of a femoral component fits or conforms to a corresponding articular surface of a tibial component. The degree of conformity depends on the shape of each articular surface and/or how the surfaces are placed relative to one another when implanted in the joint. For example, conformity may be represented by a ratio of a radius of a femoral articular surface to a radius of the corresponding tibial articular surface (e.g., 1:1.05). In one embodiment, the conformity of the prosthetic device 5 in the medial compartment can be different from the conformity in the lateral compartment. This can be accomplished by providing the surgeon with a selection of segmented components with a range of geometries (e.g., profiles, contours, dimensions, slopes, etc.). The surgeon then selects and installs components that provide the desired conformity in the medial compartment and components that provide the desired conformity in the lateral compartment.

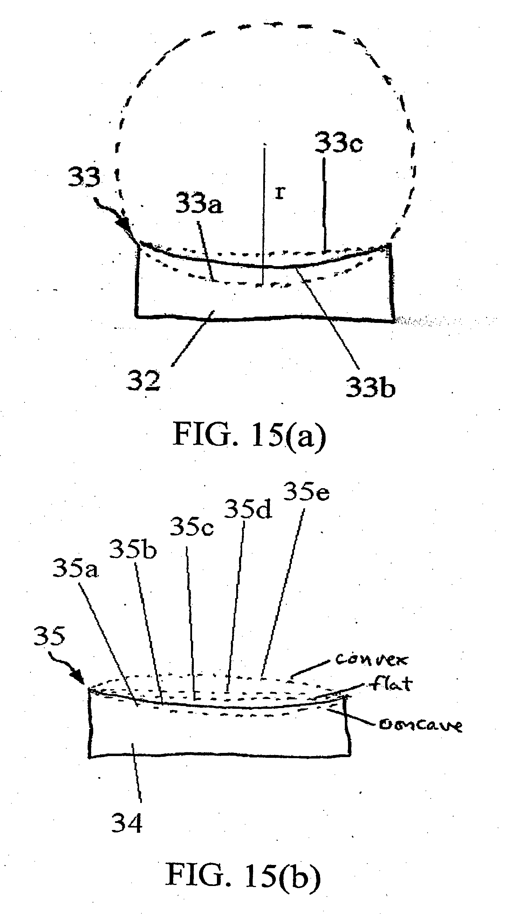

For example, in one embodiment, the prosthetic device 5 can be configured to have a first component including a first contour and a second component including a second contour. Each contour may be comprised of one or more radii and may also include substantially straight sections. As shown in FIG. 15(a), a radius of a portion of a contour is the radius r of a circle that includes the contour. The first and second contours may be any contour of a component such as, for example, a sagittal or coronal contour. The first and second contours may be similar or different. In one embodiment, as shown in FIGS. 15(a) and 15(b), the first component is a medial tibial component 32 having a first sagittal contour 33, the second component is a lateral tibial component 34 having a second sagittal contour 35. The medial tibial component 32 may be designed and manufactured with a variety of contours, such as a contour 33a, a contour 33b, and a contour 33c. Similarly, the lateral tibial component 34 may be designed and manufactured with a variety of contours, such as a contour 35a, a contour 35b, a contour 35c, a contour 35d, and a contour 35e. The contours may include any suitable shape. For example, the contours may be substantially concave (e.g., the contours 33a and 35a), substantially convex (e.g., the contour 35e), or substantially flat (e.g., the contour 35c). When the surgeon selects components for the prosthetic device 5, he can choose medial and lateral components that have similar (e.g., symmetric) or different (e.g., asymmetric) contours with the potential number of combinations limited only by the number of segmented components available. As a result, the prosthetic device 5 can be tuned or adjusted to accommodate the specific needs of each patient based on the condition of ligaments, existing anatomy, joint kinematics, range of motion, and/or desired patient outcome.

For example, the surgeon may choose components that create a prosthetic device 5 that is highly conforming in the medial compartment and mildly conforming or flat in the lateral compartment. Conversely, the prosthetic device 5 may be constructed to be mildly conforming in the medial compartment and highly conforming in the lateral compartment. Alternatively, the medial and lateral compartments may have a similar degree of conformity. In one embodiment, a medial contour is substantially concave. In another embodiment, a lateral contour is substantially less concave than a medial contour. In another embodiment, a medial contour is substantially concave, and a lateral contour is substantially flat. In another embodiment a medial contour is substantially concave, and a lateral contour is substantially convex. In a preferred embodiment, a medial contour includes a portion having a radius of between about 20 mm to about 75 mm concave. In another embodiment, a medial contour includes a portion having a radius of between about 20 mm to about 75 mm concave, and a lateral contour includes at least one of the following: (a) a portion having a radius of between about 76 mm to about 200 mm concave, (b) a portion having a radius that is greater than the medial radius, (c) a portion having a radius of between about 76 mm concave and 200 mm convex, (d) a portion having a radius that is substantially flat, and (e) a portion having a radius that is substantially flat to about 200 mm convex.

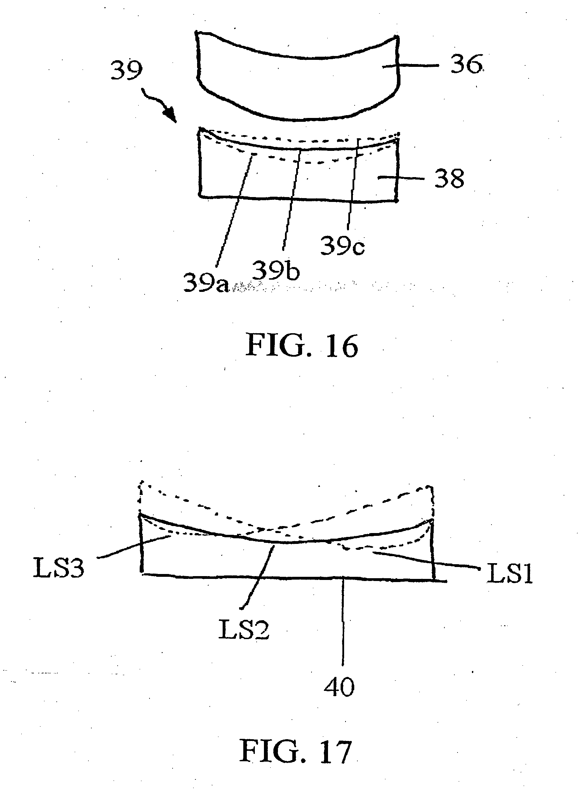

Although the embodiment of FIGS. 15(a) and 15(b) illustrates sagittal conformities, other conformities, such as coronal conformities may be adjusted in a similar manner. For example, FIG. 16 illustrates a femoral component 36 and a tibial component 38 having a contour 39. The contour 39 may be comprised of one or more radii and may also include substantially straight sections. As with sagittal contours, the component 38 may be designed and manufactured with various conformities (e.g., substantially concave, substantially flat, substantially convex, etc.) as illustrated by contours 39a, 39b, and 39c. Additionally, the prosthetic device 5 may be constructed to have medial and tibial components with similar or different coronal contours. For example, to provide medial-lateral stability, the components can be selected so that, in the coronal plane C, the coronal conformity between the femoral component 36 and the tibial component 38 can be very conforming. The tradeoff is that increased conformity results in increased constraint. By varying the components of the prosthetic device 5 the surgeon can adjust coronal conformity in one or both compartments of the joint.

The ability to vary curvature between components is advantageous. For example, in one embodiment, the shape of the surface of the tibial component can be curved to allow for controlled internal/external rotation of the femur during ROM. In another embodiment, the shape of the curve on the medial and lateral components can be selected from different components having different curves to allow for constrained motion or less constrained motion based on parameters selected by the surgeon to fit the patient anatomy and needs. In another embodiment, the coronal curvature is substantially conforming to the curvature of the femur, while the sagittal curvature is less conforming to enable additional medial-lateral stability of the joint and correct for deficient collateral ligaments. In another embodiment, the coronal curvature is mildly conforming, while the sagittal curvature is highly conforming to correct for deficient function of the cruciate ligaments that may not be severe enough to require a posterior stabilized implant.

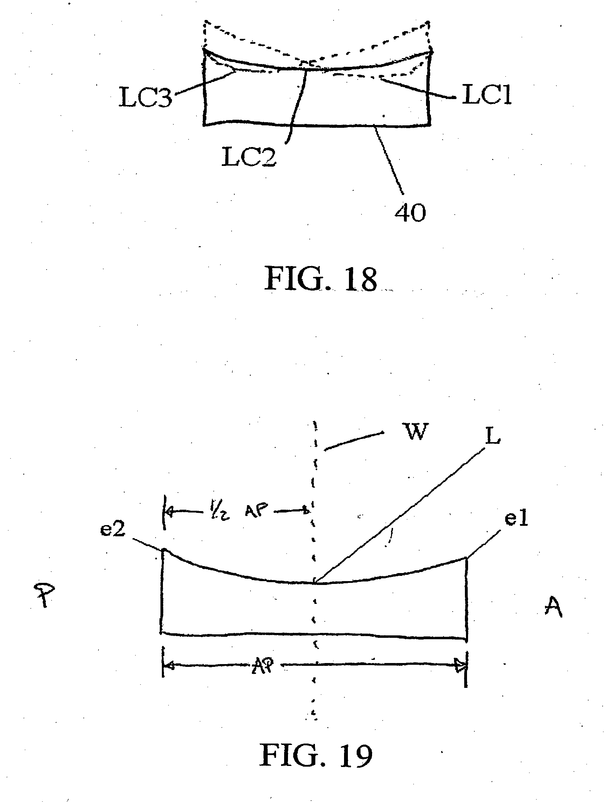

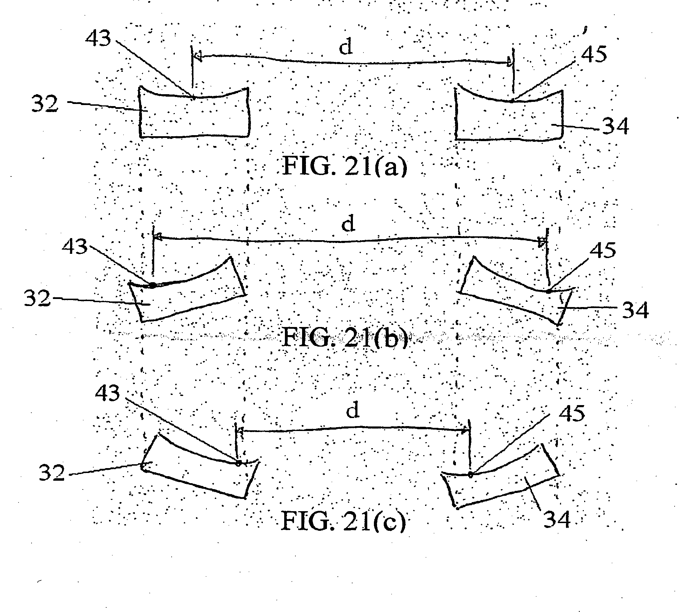

In addition to being able to vary conformities of the prosthetic device 5, medial and lateral tibial lowpoints can be varied to meet the unique stability needs of the patient and/or to match the femoral components. For example, as shown in FIG. 17, a tibial component 40 may be designed and manufactured with a variety of sagittal lowpoint Ls₁, Ls₂, and Ls₃. Similarly, as shown in FIG. 18, the tibial component 40 may be designed and manufactured with a variety of coronal lowpoints Lc₁, Lc₂, and Lc₃. In addition to providing components with shapes that have different lowpoints, lowpoint position can be adjusted by varying the orientation of the components during implantation. For example, as shown in FIGS. 21(a) to 21(c) a location of a lowpoint 43 of the medial tibial component 32, a location of a lowpoint 45 of the lateral tibial component 34, and a distance d between the lowpoints 43 and 45 can be altered by rotating or changing a slope of one or both of the components 32 and 34 during implantation.

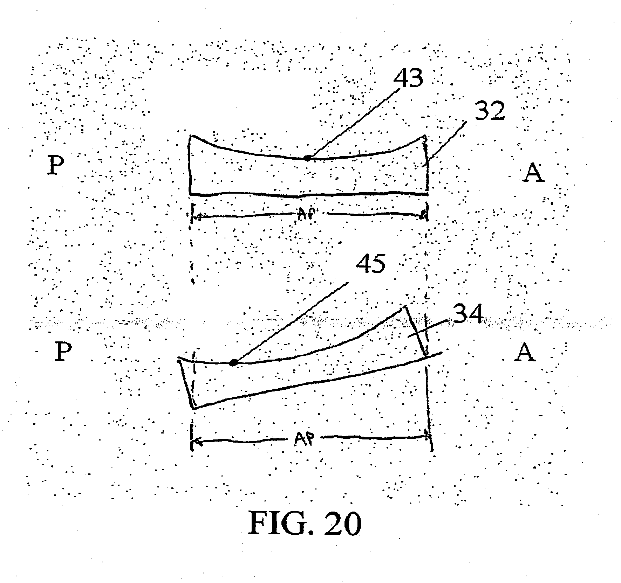

In one embodiment, the prosthetic device 5 includes a first component having a first contour with a first lowpoint and a second component having a second contour with a second lowpoint. The first and second lowpoints may have similar or different anterior-posterior (front-back) locations. In one embodiment, at least one of the first and second lowpoints (e.g., a medial lowpoints of a tibial sagittal contour) is substantially located in an anterior-posterior midplane W. FIG. 19 illustrates a lowpoint L located in the anterior-posterior midplane W, which is a plane located midway between an anterior edge e1 and a posterior edge e2 of the tibial component. In another embodiment, at least one of the first and second lowpoints (e.g., the medial lowpoint of a tibial sagittal contour) is located at a position substantially in an anterior-posterior midplane to a position 10 mm posterior to the anterior-posterior midplane. In another embodiment, the first and second components are configured to be fixed relative to the bone such that the first lowpoint and the second lowpoint (e.g., the medial and lateral lowpoints of a tibial sagittal contour) are in substantially different anterior-posterior locations. For example, in reference to FIGS. 15(a) and 15(b), the contour 33 of the medial tibial component 32 may include the lowpoint 43, and the contour 35 of the lateral tibial component 34 may include a lowpoint 45. As shown in FIG. 20, the location of the lowpoints may be changed by changing a slope of the tibial component. For example, by altering the slope of the component 34 (e.g., by tilting a posterior edge of the component 34 downward) the lowpoint 45 locates more posteriorly than the lowpoint 43 of the component 32.

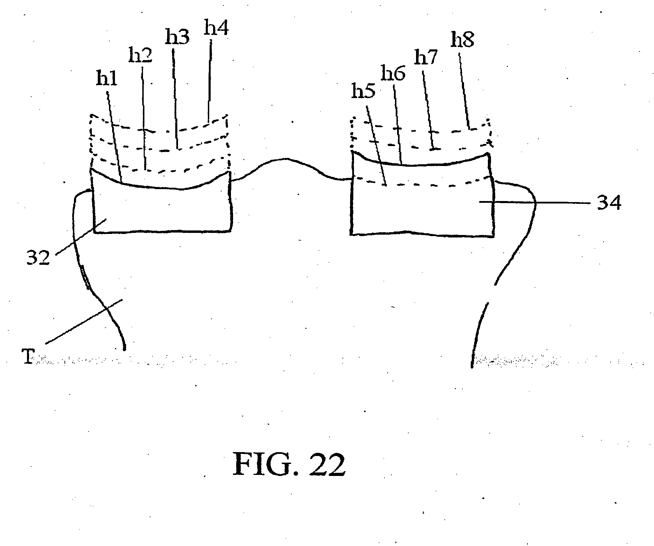

Another advantage of the segmented components of the present invention is the ability to vary tibial insert thickness to thereby adjust a height of the insert. For example, by providing tibial components of varying thicknesses and/or by placing the tibial components at different elevations on the bone, different insert heights (e.g., h1, h2, h3, h4, h5, h6, h7, h8_{;} etc.) can be achieved in the medial and lateral comportments as shown in FIG. 22. For example, after tibial and femoral bone preparation, tibial and femoral trials are positioned onto the ends of the bones. If the ligaments are too loose, a thicker insert is placed onto the tibial baseplate. If the medial compartment is balanced and the lateral side is loose, the surgeon may have to increase tibial insert thickness, release ligaments, and/or recut the tibia or femur to achieve ligament balance. For a bicondylar segmented tibial arthroplasty, only the medial and tibial compartments are resurfaced, leaving the tibial intercondylar eminence and preserving the tibial anterior and posterior cruciate ligament attachment. To achieve ligament balance, different insert thicknesses can be used in each of the medial and lateral compartments. In addition, the Hip-Knee-Ankle angle (varus/valgus) can be modified by selecting different insert thicknesses. If the leg is in varus, adding insert thickness to the medial compartment reduces the varus angle. Similarly, if the leg is in valgus, adding insert thickness to the lateral compartment reduces the valgus angle.

To install the prosthetic device 5 in the patient, the surgeon preferably uses a computer aided surgery (CAS) system to accomplish surgical planning and navigation. For example, a CAS, system may be used by the surgeon during bone preparation to achieve the desired bone resection. Preferably, the CAS system is a robotic surgical navigation system that enables the surgeon to achieve sufficient accuracy, predictability, and repeatability in planning the placement of the components of the prosthetic device 5 and in preparing the bone to receive the components. In contrast, conventional freehand and jig-based bone preparation methods may not be able to achieve sufficiently tight tolerances to enable successful installation of the prosthetic device 5.

For example, whereas conventional TKA systems comprise solid parts having fixed geometry and conventional modular systems comprise modular components that are joined together inside the body resulting in fixed geometry, the components of the prosthetic device 5 are individually positioned segmented components. Altering the placement parameters of one or more of the components results in alterations in the geometry of the prosthetic device 5. As a result, the geometry and configuration of the prosthetic device 5 are variable depending on the surgeon's placement of the segmented components relative to the patient's anatomy and/or relative to one another. To ensure that a desired placement of each component is achieved and that desired geometric relationships (e.g., distance, orientation, alignment, etc.) with the patient's anatomy and among the segmented components are established, each segmented component must be installed (or positioned) in the joint with a high degree of accuracy. Achieving the requisite accuracy requires significant surgical skill as well as specialized instruments and technology. Because surgeons have different skill levels and experience, operative results among patients may not be sufficiently predictable and/or repeatable using conventional freehand and jig-based bone preparation methods. Accordingly, in a preferred embodiment, the components of the prosthetic device 5 are configured to be fixed relative to a corresponding bone of the joint that includes at least one robotically prepared surface. The surface of the bone may be prepared, for example, as described in U.S. Patent Application Serial No. 11/357,197, filed February 21, 2006, published as Pub. No. US 2006/0142657. Additionally, relative positioning of the segmented components may be achieved, for example, using the features and techniques described in U.S. Patent Application Serial No. 11/617,449, filed December 28, 2006.

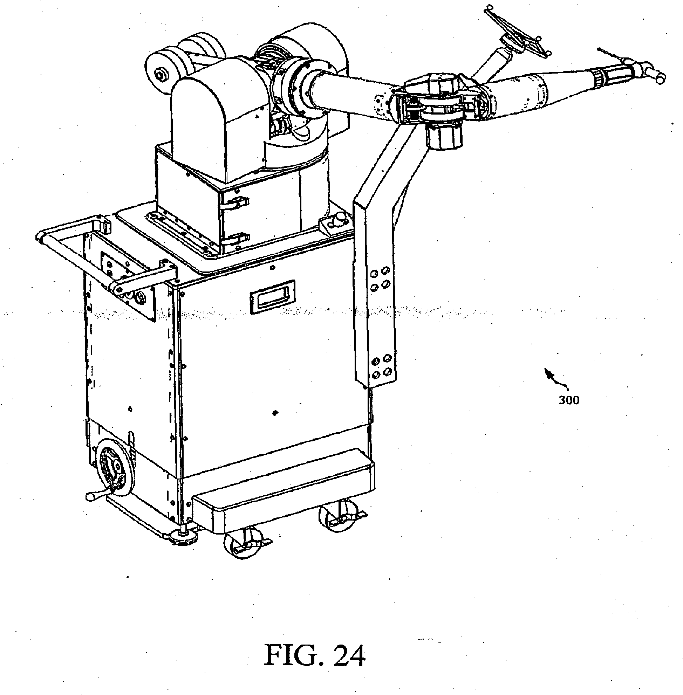

In one embodiment, the surface of the bone is prepared using a robotic surgical navigation system 300 known as the Haptic Guidance System^{™} (HGS) manufactured by MAKO Surgical Corp. and shown in FIG. 24. The surgical navigation system 300 includes a surgical planning and navigation system coupled with a haptic device that provides haptic guidance to guide the surgeon during a surgical procedure. As described in U.S. Patent Application Serial No. 11/357,197, filed February 21, 2006, published as Pub. No. US 2006/0142657, the haptic device is an interactive surgical robotic arm that holds a surgical tool (e.g., a surgical burr) and is manipulated by the surgeon to perform a procedure on the patient, such as cutting a surface of a bone in preparation for implant installation. As the surgeon manipulates the robotic arm to move the tool and sculpt the bone, the surgical navigation system 300 guides the surgeon by providing force feedback that constrains the tool from penetrating a virtual boundary. For example, the surgical tool is coupled to the robotic arm and registered to the patient's anatomy. The surgeon operates the tool by manipulating the robotic arm to move the tool and perform the cutting operation. As the surgeon cuts, the surgical navigation system 300 tracks the location of the tool and the patient's anatomy and, in most cases, allows the surgeon to freely move the tool in the workspace. However, when the tool is in proximity to the virtual boundary (which is also registered to the patient's anatomy), the surgical navigation system 300 controls the haptic device to provide haptic guidance (e.g., force feedback) that tends to constrain the surgeon from penetrating the virtual boundary with the tool.

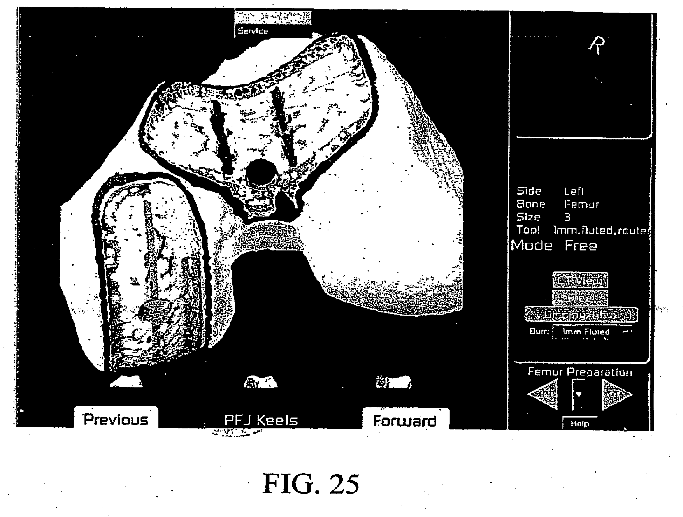

The virtual boundary may represent, for example, a cutting boundary defining a region of bone to be removed or a virtual pathway for guiding the surgical tool to a surgical site without contacting critical anatomical structures. The virtual boundary may be defined by a haptic object, and the haptic guidance may be in the form of force feedback (i.e., force and/or torque) that is mapped to the haptic object and experienced by the surgeon as resistance to further tool movement in the direction of the virtual boundary. Thus, the surgeon may feel the sensation that the tool has encountered a physical object, such as a wall. In this manner, the virtual boundary functions as a highly accurate virtual cutting guide. In one embodiment, the surgical navigation system 300 includes a visual display showing the amount of bone removed during the cutting operation as shown in FIG. 25. Because the surgical navigation system 300 utilizes tactile force feedback, the surgical navigation system 300 can supplement or replace direct visualization of the surgical site and enhance the surgeon's natural tactile sense and physical dexterity. Guidance from the haptic device coupled with computer aided surgery, enables the surgeon to actively and accurately control surgical actions (e.g., bone cutting) to achieve the tolerances and complex bone resection shapes that enable optimal and customized installation of the components of the prosthetic device 5.

In addition to bone preparation, a CAS system enables the surgeon to customize the placement of the components to construct a prosthetic device tailored to the specific needs of the patient based on the patient's unique anatomy, ligament stability, kinematics, and/or disease state. Implant planning may be accomplished preoperatively or intraoperatively and may be evaluated and adjusted in real time during execution of the surgical procedure. In a preferred embodiment, implant planning is accomplished using the surgical navigation system 300 known as the Haptic Guidance System™ (HGS) manufactured by MAKO Surgical Corp. and as described in U.S. Patent Application Serial No. 11/357,197, filed February 21, 2006, published as Pub. No. US 2006/0142657. For example, the surgeon may use the surgical planning features of the surgical navigation system 300 to plan the placement of each component relative to a preoperative CT image (or other image or model of the anatomy). The software enables the surgeon to view the placement of each component relative to the anatomy and to other components. The software may also be configured to illustrate how the components will interact as the joint moves through a range of motion. Based on the component placement selected by the surgeon, the surgical navigation system 300 software generates one or more haptic objects, which create one or more virtual boundaries representing, for example, a portion of bone to be removed or critical anatomy to be avoided. During surgery, the haptic object is registered to the patient's anatomy. By providing force feedback, the surgical navigation system 300 enables the surgeon in interact with the haptic object in the virtual environment. In this manner, the surgical navigation system 300 haptically guides the surgeon during bone preparation to sculpt or contour the appropriate location of the bone so that a shape of the bone substantially conforms to a shape of a mating surface of a component of the prosthetic device 5.

In a preferred embodiment, the surgical navigation system 300 is used by the surgeon to preoperatively plan implant placement using computer simulation tools to determine whether the preoperative plan will result in the desired clinical results. Then, during surgery, the surgeon may query the soft tissue and ligaments during range of motion using appropriate instrumentation and sensors as is well known. This information may be combined with the computer simulation information of the surgical navigation system 300 to adjust the implant planning and suggest to the surgeon potential changes and adjustments to implant placement that may achieve the desired clinical outcomes.

According to one example, a surgical method of implanting the prosthetic device 5 comprises steps S1 to S4. In step S1, the surgeons selects a first component configured to be implanted in a body. In step S2, the surgeon determines a placement at which the first component will be fixed relative to a bone of the body. In step S3, the surgeon selects a second component configured to be implanted in the body. In step S4, the surgeon determines a placement at which the second component will be fixed relative to the bone. The determination of the placement of the second component is not constrained by a connection to the first component. The method of this example may further include one or more of steps S5 to S11.

In step S5, at least one of a geometry, a conformity, and a configuration of the prosthetic device is varied by varying at least one of the selection of the first component, the selection of the second component, the placement of the first component, and the placement of the second component. In step S6, the first and second components are placed relative to the bone where an alignment of the second component is not determinative of an alignment of the first component, the degrees of freedom of the second component are not determinative of the degrees of freedom of the first component, and/or the selection of the first component is not determinative of the selection of the second component. In step S7, the first and second components are implanted so that they are not connected. In step S8, the first and second components are implanted so that they are not in contact. In step S9, the first component and the second component are each affixed only to an anatomy (e.g., bone) of the patient and not to one another. The first and second components may be affixed to the anatomy in any known manner such as a press fit, a fastener, an intramedullary rod, cement, an adhesive, biological in-growth, and the like. In step S10, the surgeon selects a third component configured to be implanted in the body. In step S11, the surgeon determines a placement at which the third component will be fixed relative to the bone. The surgeon's determination of the placement of the third component is not constrained by a connection of the third component to the first component or the second component. Additionally, the selection of the first component and the selection of the second component are not determinative of the selection of the third component.

The surgical method described is intended as an exemplary illustration only. In other examples, the order of the steps of the method may be rearranged in any manner suitable for a particular surgical application. Additionally, other examples may include all, some, or only portions of the steps of the surgical method and may combine the steps of the method with existing and/or later developed surgical approaches.

Thus, according to embodiments of the present invention, an orthopedic joint prosthesis and techniques that enable customization of implant fit and performance based on each patient's unique anatomy, ligament stability, kinematics, and/or disease state are provided.

Other embodiments of the invention will be apparent to those skilled in the art from consideration of the specification and practice of the invention disclosed herein. It is intended that the specification and examples be considered as exemplary only.

## Claims

1. A method of planning implant placement of a prosthetic device (5) configured to form at least a portion of a joint, the method comprising the steps of:
selecting a first femoral or tibial component (14, 15) of the prosthetic device configured to be implanted on a central region of a femur (230) or tibia (240);
determining a placement at which the first femoral or tibial component (14, 15) will be fixed relative to the femur (230) or tibia (240);
selecting a second femoral or tibial component (10, 12, 11, 13) of the prosthetic device (5) configured to be implanted on at least one of a lateral region and a medial region of the femur (230) or tibia (240); and
determining a placement at which the second femoral or tibial component (10, 12, 11, 13) will be fixed relative to the femur (230) or tibia (240),
wherein the determination of the placement of the second component (10, 12, 11, 13) is not constrained by a connection to the first component (14, 15), and
wherein the first femoral component (14) includes a feature (20) configured to constrain a portion of the first tibial component (15) implanted on a central region of a tibia (240) and wherein the first tibial component (15) includes a feature (22) configured to constrain movement of the first tibial component (15) relative to at least a portion of the first femoral component (14) implanted on a central region of the femur (230).

2. The method of claim 1, wherein:
the first tibial component is a first segmented tibial component (38) of the prosthetic device (5), the first segmented tibial component (38) including a first contour having a first lowpoint;
the step of determining a placement at which the first component (38) will be fixed relative to the femur (230) or tibia (240) includes determining a placement at which the first segmented tibial component (38) will be fixed relative to the tibia (240);
the second component is a second segmented tibial component (32, 34) including a second contour having a second lowpoint (43, 45); and
wherein the placement of the first segmented tibial component (38) and the placement of the second segmented tibial component (32, 34) are selected such that the first and second lowpoints have different anterior-posterior locations when the first and second segmented tibial components (38, 32, 34) are implanted on the tibia (240).

3. A prosthetic device (5) configured to form at least a portion of a joint, comprising:
a plurality of components configured to be implanted in a body including at least one of multiple femoral components to be implanted on a femur and multiple tibial components to be implanted on a tibia, the plurality of components including
a femoral component (14) configured to be implanted on a central region of the femur (230), and
a tibial component (15) configured to be implanted on a central region of the tibia (240),
wherein the femoral component (14) configured to be implanted on the central region of the femur (230) includes a feature (20) configured to constrain a portion of the tibial component (15) configured to be implanted on the central region of the tibia (240),
wherein the tibial component (15) configured to be implanted on the central region of the tibia (240) includes a feature (22) configured to constrain movement of the tibial component (15) configured to be implanted on the central region of the tibia (240) relative to at least a portion of the femoral component (14) configured to be implanted on the central region of the femur (230), and
wherein each of the plurality of components is configured to be fixed relative to a bone of the body and each of the plurality of components is configured such that a placement at which the component will be fixed relative to the bone is not constrained by a connection to another of the components that is fixed relative to the same bone.

4. The prosthetic device (5) of claim 3, wherein the components are configured such that the components can be implanted to form the prosthetic device (5) without being in contact.

5. The prosthetic device (5) of claim 3, wherein the feature for constraining a portion of the tibial component includes a stop member (20b).

6. The prosthetic device (5) of claim 5, wherein the stop member (20b) includes at least one surface of a recess (20a).

7. The prosthetic device (5) of claim 3, wherein the feature configured to constrain movement of the tibial component configured to be implanted on a central region of the tibia includes a projection configured to contact a stop member (20b) disposed on the femoral component (14).

8. The prosthetic device (5) of claim 7, wherein the projection is configured so that at least one of an anterior, a posterior, a medial, or a lateral region of the projection contacts the stop member (20b).

9. The prosthetic device (5) of claim 3, wherein the plurality of components include a plurality of segmented components configured to form at least a portion of a joint and including a first segmented tibial component (38) including a first contour (39) and a second segmented tibial component (32, 34) including a second contour,
wherein the first contour (39) includes a first lowpoint and the second contour includes a second lowpoint (43, 45), the first and second lowpoints being configured to have different anterior-posterior locations when implanted on a bone of a patient, and
wherein each of the plurality of segmented components (38, 32, 34) is configured such that a placement of one of the segmented components in the joint is not constrained by a connection to another of the segmented components.

10. The prosthetic device (5) of claim 9, wherein the first contour and the second contour are asymmetric.

11. The prosthetic device (5) of claim 9, wherein the first and second contours are sagittal contours or coronal contours.

12. The prosthetic device (5) of claim 9, wherein the second contour is substantially less concave than the first contour.

13. The prosthetic device (5) of claim 9, wherein the first contour includes a portion including a radius of between about 20 to about 75 mm concave, and the second contour includes a portion including at least one of a radius of between about 76 to about 200 mm concave, a radius greater than the radius of the first contour, and a radius of between about 76 mm concave to about 200 mm concave.

14. The prosthetic device (5) of claim 9, wherein the first contour includes a concave portion and the second contour includes a flat portion.

15. The prosthetic device (5) of claim 9, wherein the first segmented tibial component (38) is configured to be fixed relative to a bone of the joint such that the first lowpoint is located at a position substantially in an anterior-posterior midplane to 10 mm posterior to the anterior-posterior midplane.

## Patentansprüche

1. Verfahren zur geplanten Platzierung einer Prothese (5), die derart konfiguriert ist, um mindestens einen Anteil von einem Gelenk zu bilden, wobei das Verfahren folgende Schritte umfasst:
Auswählen eines ersten Oberschenkel- oder Schienbeinbestandteils (14, 15) der Prothese, der derart konfiguriert ist, dass er in einen zentralen Bereich eines Oberschenkels (230) oder eines Schienbeins (240) implantiert werden kann;
Bestimmen einer Platzierung, bei der der erste Oberschenkel- oder Schienbeinbestandteil (14,15) relativ zum Oberschenkel (230) oder Schienbein (240) befestigt wird;
Auswählen eines zweiten Oberschenkel- oder Schienbeinbestandteils (10, 12,11, 13) der Prothese (5), der derart konfiguriert ist, dass er in mindestens einem lateralen Bereich und einem medizinischen Bereich des Oberschenkels (230) oder Schienbeins (240) implantiert werden kann und
Bestimmen einer Platzierung, bei der der zweite Oberschenkel- oder Schienbeinbestandteil (10, 12,11, 13) relativ zum Oberschenkel (230) oder Schienbein (240) befestigt wird,
wobei die Bestimmung der Platzierung des zweiten Bestandteils (10, 12,11, 13) nicht durch eine Verbindung zum ersten Bestandteil (14, 15) eingeschränkt wird und
wobei der erste Oberschenkelbestandteil (14) eine Einrichtung (20) umfasst, die derart konfiguriert ist, dass sie einen Anteil des ersten Schienbeinbestandteils (15), der in einem zentralen Bereich des Schienbeins (240) implantiert ist, beschränkt und wobei der erste Schienbeinbestandteil (15) eine Einrichtung (22) umfasst, die derart konfiguriert ist, dass sie die Bewegung des ersten Schienbeinbestandteils (15) relativ zu mindestens einem Anteil des ersten Oberschenkelbestandteils (14), der im zentralen Bereich des Oberschenkels (230) implantiert ist, beschränkt.

2. Verfahren nach Anspruch 1, wobei
der erste Schienbeinbestandteil einen ersten mehrteiligen Schienbeinbestandteil (38) der Prothese (5) darstellt und der erste mehrteilige (38) Schienbeinbestandteil ein erstes Profil umfasst, was einen ersten Tiefpunkt hat.
Der Schritt des Bestimmens einer Platzierung, bei der der erste Bestandteil (38) relativ zum Oberschenkel (230) oder Schienbein (240) befestigt wird, umfasst ein Bestimmens einer Platzierung, bei der der erste mehrteilige Schienbeinbestandteil (38) relativ zum Schienbein (240) befestigt wird;
der zweite Bestandteil einen zweiten mehrteiligen Schienbeinbestandteil (32, 34) darstellt, das ein zweites Profil umfasst, was einen zweiten Tiefpunkt (43, 45) hat, und
wobei die Platzierung des ersten mehrteiligen Schienbeinbestandteil (38) und die Platzierung des zweiten mehrteiligen Schienbeinbestandteil (32, 34) derart ausgewählt werden, so dass die ersten und zweiten Tiefpunkte unterschiedliche vordere und hintere Standorte haben, wenn das erste und das zweite mehrteilige Schienbeinbestandteil (38, 32, 34) auf das Schienbein (240) implantiert wird.

3. Prothese (5), die derart konfiguriert ist, um mindestens einen Anteil eines Gelenks zu bilden, was Folgendes umfasst:
Eine Mehrzahl von Bestandteilen, die für die Implantation in einem Körper konfiguriert sind, die mindestens eine der mehrteiligen Oberschenkelbestandteile umfasst, die auf den Oberschenkel implantiert wird, und mehrteilige Schienbeinbestandteile umfasst, die auf das Schienbein implantiert werden, wobei die Mehrzahl der Bestandteile folgendes umfasst:
ein Oberschenkelbestandteil (14), der derart konfiguriert ist, dass er in einen zentralen Bereich eines Oberschenkels (230) implantiert werden kann;
ein Schienbeinbestandteil (15), der derart konfiguriert ist, dass er in einen zentralen Bereich eines Schienbeins (240) implantiert werden kann;
wobei der Oberschenkelbestandteil (14), der derart konfiguriert ist, dass er in einen zentralen Bereich eines Oberschenkels (230) implantiert werden kann, eine Einrichtung (20) umfasst, die derart konfiguriert ist, dass sie einen Teil des Oberschenkelbestandteils (15) einschränkt, der derart konfiguriert ist, dass er in einen zentralen Bereich eines Oberschenkels (240) implantiert werden kann ,
wobei der Oberschenkelbestandteil (15) konfiguriert ist, in einem zentralen Bereich des Oberschenkels (240) konfiguriert zu werden, eine Einrichtung (22) umfasst, die derart konfiguriert ist, dass sie die Bewegung des ersten Schienbeinbestandteils (15), der für die Implantation in einem zentralen Bereich des Schienbeins (240) konfiguriert ist, relativ zu mindestens einem Anteil des Oberschenkelbestandteils (14) beschränkt, der für die Implantation im zentralen Bereich des Oberschenkels (230) konfiguriert ist, und
wobei jeder der Mehrzahl von Bestandteilen derart konfiguriert ist, um relativ zu einem Knochen im Körper befestigt zu werden, und jeder der Mehrzahl von Bestandteilen derart konfiguriert ist, dass eine Platzierung, bei der das Bestandteil relativ zum Knochen befestigt wird, nicht durch eine Verbindung zu einem anderen Bestandteil, das relativ zu dem gleichen Knochen befestigt wird, eingeschränkt wird.

4. Prothese (5) nach Anspruch 3, wobei die Bestandteile derart konfiguriert sind, dass die Bestandteile implantiert werden können, um eine Prothese (5) zu bilden, ohne dabei in Kontakt zu treten.

5. Prothese (5) nach Anspruch 3, wobei die Einrichtung für die Beschränkung eines Anteils des Schienbeinbestandteils ein Stoppelement (20b) umfasst.

6. Prothese (5) nach Anspruch 5, wobei das Stoppelement (20b) mindestens eine Kerbenoberfläche (20a) umfasst.

7. Prothese (5) nach Anspruch 3, wobei die Einrichtung, die derart konfiguriert ist, dass sie die Bewegung des Schienbeinbestandteils, der für die Implantation in einem zentralen Bereich des Schienbeins konfiguriert ist, beschränkt, eine Erhebung umfasst, die derart konfiguriert ist, um mit einem Stoppelement (20b) Kontakt aufzunehmen, das auf dem Oberschenkelbestandteil (14) angeordnet ist.

8. Prothese (5) nach Anspruch 7, wobei die Erhebung derart konfiguriert ist, dass mindestens einer der vorderen, hinteren, medialen oder lateralen Bereiche der Erhebung mit einem Stoppelement (20b) Kontakt aufnimmt.

9. Prothese (5) nach Anspruch 3, wobei die Mehrzahl von Bestandteilen eine Mehrzahl von mehrteiligen Bestandteilen umfasst, die derart konfiguriert sind, um mindestens einen Anteil von einem Gelenk zu bilden, das ein erstes mehrteiliges Schienbeinbestandteil (38) mit einem ersten Profil (39) und ein zweites mehrteiliges Schienbeinbestandteil (32, 34) mit einem zweiten Profil umfasst,
wobei das erste Profil (39) einen ersten Tiefpunkt und das zweite Profil einen zweiten Tiefpunkt (43, 45) umfasst, wobei der erste und zweite Tiefpunkt derart konfiguriert sind, um unterschiedliche vordere und hintere Standorte aufzuweisen, wenn sie auf dem Knochen eines Patienten implantiert werden und
jeder der Mehrzahl von mehrteiligen Bestandteilen (38, 32, 34) derart konfiguriert ist, dass eine Platzierung eines der mehrteiligen Bestandteile im Gelenk nicht durch eine Verbindung mit einem anderen der mehrteiligen Bestandteile beschränkt wird.

10. Prothese (5) nach Anspruch 9, wobei das erste Profil und das zweite Profil asymmetrisch sind.

11. Prothese (5) nach Anspruch 9, wobei das erste Profil und das zweite Profil pfeilrechte und koronale Profile darstellen.

12. Prothese (5) nach Anspruch 9, wobei das zweite Profil wesentlich weniger konkav als das erste Profil ist.

13. Prothese (5) nach Anspruch 9, wobei das erste Profil einen Anteil umfasst, der mindestens einen konkaven Radius zwischen ungefähr 20 und ungefähr 75 Millimeter umfasst, und das zweite Profil einen Anteil umfasst, der mindestens einen konkaven Radius zwischen ungefähr 76 und ungefähr 200 Millimeter, also einen größeren Radius als der Radius des ersten Profils, und einen konkaven Radius zwischen ungefähr 76 und ungefähr 200 Millimeter umfasst.

14. Prothese (5) nach Anspruch 9, wobei das erste Profil einen konkaven Anteil und das zweite Profil einen flachen Anteil umfasst.

15. Prothese (5) nach Anspruch 9, wobei der erste mehrteilige Schienbeinbestandteil (38) derart konfiguriert ist, dass er relativ zu einem Knochen von einem Gelenk befestigt wird, so dass der erste Tiefpunkt sich bei einer Position befindet, die wesentlich auf einer vorderen und hinteren Mittelebene ist, die 10 Millimeter vor der vorderen und hinteren Mittelebene ist.

## Revendications

1. Procédé de planification du placement d'implant d'un dispositif prothétique (5) configuré pour former au moins une partie d'une articulation, le procédé comprenant les étapes suivantes :
sélection d'un premier composant fémoral ou tibial (14, 15) du dispositif prothétique configuré pour être implanté sur une région centrale d'un fémur (230) ou d'un tibia (240) ;
détermination d'une position à laquelle le premier composant fémoral ou tibial (14, 15) sera fixé par rapport au fémur (230) ou au tibia (240) ;
sélection d'un deuxième composant fémoral ou tibial (10, 12, 11, 13) du dispositif prothétique (5) configuré pour être implanté sur au moins une région latérale et une région médiale du fémur (230) ou du tibia (240) ; et
détermination d'une position à laquelle le deuxième composant fémoral ou tibial (10, 12, 11, 13) sera fixé par rapport au fémur (230) ou au tibia (240) ;
dans lequel la détermination du placement du deuxième composant (10, 12, 11, 13) n'est pas contrainte par une connexion au premier composant (14, 15), et
dans lequel le premier composant fémoral (14) comprend un élément (20) configuré pour contraindre une partie du premier composant tibial (15) implantée sur une région centrale d'un tibia (240) et dans lequel le premier composant tibial (15) comprend un élément (22) configuré pour contraindre le mouvement du premier composant tibial (15) par rapport à au moins une partie du premier composant fémoral (14) implantée sur une région centrale du fémur (230).

2. Procédé de la revendication 1, dans lequel :
le premier composant tibial est un premier composant tibial segmenté (38) du dispositif prothétique (5), le premier composant tibial segmenté (38) comprenant un premier contour ayant un premier point bas ;
l'étape de détermination d'une position dans laquelle le premier composant (38) sera fixé par rapport au fémur (230) ou au tibia (240) comprend la détermination d'une position à laquelle le premier composant tibial segmenté (38) sera fixé par rapport au tibia (240) ;
le deuxième composant est un deuxième composant tibial segmenté (32, 34) comprenant un deuxième contour ayant un deuxième point bas (43, 45) ; et
la position du premier composant tibial segmenté (38) et la position du deuxième composant tibial segmenté (32, 34) sont sélectionnées de telle sorte que le premier et le deuxième point bas présentent des localisations antérieure-postérieure différentes lorsque le premier et le deuxième composant tibial segmenté (38, 32, 34) sont implantés sur le tibia (240).

3. Dispositif prothétique (5) configuré pour former au moins une portion d'une articulation, comprenant :
une pluralité de composants configurés pour être implantés dans un corps comprenant au moins un parmi de multiple composants fémoraux à implanter sur un fémur et de multiples composants tibiaux à implanter sur un tibia, la pluralité de composants comprenant
un composant fémoral (14) configuré pour être implanté sur une région centrale du fémur (230), et
un composant tibial (15) configuré pour être implanté sur une région centrale du tibia (240),
dans lequel le composant fémoral (14) configuré pour être implanté sur la région centrale du fémur (230) comprend un élément (20) configuré pour contraindre une partie du composant tibial (15) configurée pour être implanté sur la région centrale du tibia (240),
dans lequel le composant tibial (15) configuré pour être implanté sur la région centrale du tibia (240) comprend un élément (22) configuré pour contraindre un mouvement du composant tibial (15) configuré pour être implanté sur la région centrale du tibia (240) par rapport à au moins une partie du premier composant fémoral (14) configuré pour être implanté sur la région centrale du fémur (230), et
chaque composant de la pluralité de composants est configuré pour être fixé par rapport à un os du corps et chaque composant de la pluralité de composants est configuré pour qu'une position à laquelle le composant sera fixé par rapport à l'os ne soit pas contrainte par une connexion à un autre des composants qui est fixé par rapport au même os.

4. Dispositif prothétique (5) de la revendication 3, dans lequel les composants sont configurés pour pouvoir être implantés afin de former le dispositif prothétique (5) sans être en contact.

5. Dispositif prothétique (5) de la revendication 3, dans lequel l'élément destiné à contraindre une portion du composant tibial comprend un élément d'arrêt (20b).

6. Dispositif prothétique (5) de la revendication 5, dans lequel l'élément d'arrêt (20b) comprend au moins une surface d'un évidemment (20a).

7. Dispositif prothétique (5) de la revendication 3, dans lequel l'élément configuré pour contraindre le mouvement du composant tibial configuré pour être implanté sur une région centrale du tibia comprend une projection configurée pour entrer en contact avec un élément d'arrêt (20b) placé sur le composant fémoral (14).

8. Dispositif prothétique (5) de la revendication 7, dans lequel la projection est configurée pour qu'au moins une parmi une région antérieure, une région postérieure, une région médiale ou une région latérale de la projection entre en contact avec l'élément d'arrêt (20b).

9. Dispositif prothétique (5) de la revendication 3, dans lequel la pluralité de composants comprend une pluralité de composants segmentés configurés pour former au moins une portion d'une articulation et incluant un premier composant tibial segmenté (38) comprenant un premier contour (39) et un deuxième composant tibial segmenté (32, 34) comprenant un deuxième contour,
le premier contour (39) comprenant un premier point bas et le deuxième contour comprenant un deuxième point bas (43, 45), le premier et le deuxième point bas étant configurés pour présenter des localisations antérieure-postérieure différentes lors de l'implantation sur un os d'un patient, et
dans lequel chaque composant de la pluralité de composants segmentés (38, 32, 34) est configuré pour qu'une position d'un des composants segmentés dans l'articulation ne soit pas contrainte par une connexion à un autre des composants segmentés.

10. Dispositif prothétique (5) de la revendication 9, dans lequel le premier contour et le deuxième contour sont asymétriques.

11. Dispositif prothétique (5) de la revendication 9, dans lequel le premier contour et le contour sont des contours sagittaux ou des contours coronaux.

12. Dispositif prothétique (5) de la revendication 9, dans lequel le deuxième contour est sensiblement moins concave que le premier contour.

13. Dispositif prothétique (5) de la revendication 9, dans lequel le premier contour comprend une portion comprenant un rayon compris entre environ 20 et environ 75 mm concave, et le deuxième contour comprend une portion comprenant au moins un rayon compris entre environ 76 et environ 200 mm concave, un rayon supérieur au rayon du premier contour et un rayon compris entre environ 76 mm concave et environ 200 mm concave.

14. Dispositif prothétique (5) de la revendication 9, dans lequel le premier contour comprend une portion concave et le deuxième contour comprend une portion plate.

15. Dispositif prothétique (5) de la revendication 9, dans lequel le premier composant tibial segmenté (38) est configuré pour être fixé par rapport à un os de l'articulation de telle sorte que le premier point bas soit situé en une position sensiblement dans un plan moyen antérieur-postérieur à 10 mm postérieur au plan moyen antérieur-postérieur.
